# EUROPEAN PATENT APPLICATION

(11) **EP 4 276 110 A1**
(43) Date of publication of application: **15.11.2023**
(21) Application number: 22736572.3
(22) Date of filing: 07.01.2022
(51) Int. Cl.: C07K 16/28, C12N 15/13, C12N 15/63, A61K 39/395, A61P 35/00

(54) **ANTI-PD-L1/ANTI-4-1BB NATURAL ANTIBODY STRUCTURE-LIKE HETERODIMERIC FORM BISPECIFIC ANTIBODY AND PREPARATION THEREOF**

(30) Priority: 08.01.2021 CN 202110025200
(71) Applicant: Beijing Hanmi Pharmaceutical Co., Ltd., Beijing 101312 (CN)
(72) Inventor: LIU, Jiawang, Beijing 101312 (CN); YANG, Yaping, Beijing 101312 (CN); ZHAO, Siqi, Beijing 101312 (CN); LIU, Yang, Beijing 101312 (CN); SONG, Nanmeng, Beijing 101312 (CN); FAN, Fei, Beijing 101312 (CN); SU, Kaixuan, Beijing 101312 (CN); ZHANG, Lanxin, Beijing 101312 (CN); WANG, Jing, Beijing 101312 (CN); XU, Jiangcheng, Beijing 101312 (CN); LEE, Kyoung Woo, Beijing 101312 (CN)
(74) Representative: Høiberg P/S
(86) International application number: PCT/CN2022/070624
(87) International publication number: WO 2022/148410

(57) **Abstract**

Provided are an anti-PD-L1/anti-4-lBB natural antibody structure-like heterodimeric form bispecific antibody and a preparation thereof. Specifically, provided are an anti-PD-L1/anti-4-1BB bispecific antibody that has natural IgG features, that has no mismatch between heavy and light chains and that is in the form of a highly stable heterodimer, as well as a preparation method therefor. The bispecific antibody can simultaneously bind two target molecules and is more effective in treating complex diseases and has fewer side effects.

## Description

### Field of the Invention

The present invention relates to an anti-PD-L1/anti-4-1BB bispecific antibody in the form of a natural antibody structure-like heterodimer and preparation thereof. In particular, the present invention provides an anti-PD-L1/anti-4-1BB bispecific antibody in the form of a highly stable heterodimer with the characteristics of natural IgGs and no heavy and light chain mismatches, as well as the preparation method thereof.

### Background of the Invention

Programmed death ligand-1 (PD-L1) is a ligand for immune checkpoint programmed death receptor-1 (programmed death-1, PD-1). PD-L1 belongs to the B7 family and is expressed in an inducible manner on the surface of a variety of immune cells, including T cells, B cells, monocytes, macrophages, DC cells, endothelial cells, epidermal cells, etc. After binding to PD-1, PD-L1 is mainly involved in the negative regulation of T cell activation, and is able to regulate the strength and duration of an immune response. In addition to acting as a ligand for PD-1, PD-L1 can also act as a ligand for CD80, to deliver negative regulatory signals to T cells and to induce T cell immune tolerance (Autoimmun Rev, 2013, 12(11): 1091-1100.; Front Immunol, 2013, 4: 481.; Nat Rev Cancer, 2012, 12(4): 252-264.; Trends Mol Med. 2015 Jan;21(1):24-33.; Clin Cancer Res. 2012 Dec 15;18(24):6580-7.). Normally, PD-L1 and PD-1 can mediate and maintain body's autoimmune tolerance, prevent the excessive activation of immune system from damaging its own tissues during an inflammatory reaction, and thus play a positive role in preventing the occurrence of an autoimmune disease. Under pathological conditions, PD-L1 and PD-1 are involved in tumor immunity as well as the occurrence and development of various autoimmune diseases. Several studies have reported that PD-L1 is highly expressed in a variety of tumor tissues, PD-1 is highly expressed in tumor infiltrating lymphocytes, and the overexpressions of PD-L1 and PD-1 are tightly associated with poor clinical prognosis of tumors (Anticancer Agents Med Chem. 2015;15(3):307-13.; Hematol Oncol Stem Cell Ther. 2014 Mar;7(1):1-17.; Trends Mol Med. 2015 Jan;21(1):24-33.; Immunity. 2013 Jul 25;39(1):61-73. J Clin Oncol. 2015 Jun 10;33(17):1974-82.). Blocking the interactions of PD-L1/PD-1 and CD80/PD-L1 with a PD-L1 monoclonal antibody has shown good anti-tumor effects in both preclinical experimental studies and in clinical trials. Currently, anti-PD-L1 monoclonal antibody has been approved for the treatment of various tumors such as non-small cell lung cancer and urothelial carcinoma.

4-1BB (also known as CD137, TNFRSF9 etc.) is a transmembrane protein of the tumor necrosis factor receptor superfamily (TNFRSF). 4-1BB is expressed on DCs, activated monocytes, NK cells, neutrophils, eosinophils and mast cells. 4-1BBL (CD137L) is a glycoprotein member of the TNF superfamily and is mainly expressed on activated B cells, macrophages, dendritic cells, and myeloid cells. 4-1BB is a costimulatory molecule on CD8⁺- and CD4⁺- T cells, regulatory T cells (Tregs), natural killer T cells (NK(T) cells), B cells and neutrophils. The binding of 4-1BB to 4-1BBL activates intracellular signaling pathways. Studies have shown that in many models, some 4-1BB agonistic mAbs increase the expression of the costimulatory molecules, and significantly enhance the cytolytic T lymphocyte response, resulting in anti-tumor efficacy.

Therefore, there is still a need in the present field to develop a novel therapeutic drug that binds to the PD-L1 and 4-1BB simultaneously.

### Summary of the Invention

The present invention provides a novel bifunctional antibody in the form of a highly stable heterodimer with the characteristics of natural IgGs and no heavy and light chain mismatches and capable of binding to PD-L1 and 4-1BB simultaneously and the preparation method thereof. The bifunctional antibody tends to bind to the tumor cells highly expressing PD-L1 and the T cell expressing 4-1BB simultaneously, and thus exerts efficient and specific killing effects with lower toxicity and side effects.

One aspect of the present invention relates to a bispecific antibody comprising a first antigen-binding functional region that specifically binds to PD-L1 and a second antigen-binding functional region that specifically binds to 4-1BB, wherein the first antigen-binding functional region that specifically binds PD-L1 comprises:
(A) a heavy chain variable region, comprising
   (a) HCDR1, which comprises the amino acid sequence set forth in SEQ ID NO: 15,
   (b) HCDR2, which comprises the amino acid sequence set forth in SEQ ID NO: 16, and
   (c) HCDR3, which comprises the amino acid sequence set forth in SEQ ID NO: 17; and
(B) a light chain variable region, comprising
   (a) LCDR1, which comprises the amino acid sequence set forth in SEQ ID NO: 18,
   (b) LCDR2, which comprises the amino acid sequence set forth in SEQ ID NO: 19, and
   (c) LCDR3, which comprises the amino acid sequence set forth in SEQ ID NO: 20.

In some embodiments, the second antigen-binding functional region of the bispecific antibody that specifically binds to 4-1BB comprises
(A) a heavy chain variable region, comprising
   (a) HCDR1, which comprises the amino acid sequence set forth in SEQ ID NO: 21,
   (b) HCDR2, which comprises the amino acid sequence set forth in SEQ ID NO: 22, and
   (c) HCDR3, which comprises the amino acid sequence set forth in SEQ ID NO: 23; and
(B) a light chain variable region, comprising
   (a) LCDR1, which comprises the amino acid sequence set forth in SEQ ID NO: 24,
   (b) LCDR2, which comprises the amino acid sequence set forth in SEQ ID NO: 25, and
   (c) LCDR3, which comprises the amino acid sequence set forth in SEQ ID NO: 26.

In some embodiments, the first antigen-binding functional region of the bispecific antibody that specifically binds to PD-L1 comprises
(A) a heavy chain variable region, comprising
   (a) HCDR1, which comprises the amino acid sequence set forth in SEQ ID NO: 15,
   (b) HCDR2, which comprises the amino acid sequence set forth in SEQ ID NO: 16, and
   (c) HCDR3, which comprises the amino acid sequence set forth in SEQ ID NO: 17; and
(B) a light chain variable region, comprising
   (a) LCDR1, which comprises the amino acid sequence set forth in SEQ ID NO: 18,
   (b) LCDR2, which comprises the amino acid sequence set forth in SEQ ID NO: 19, and
   (c) LCDR3, which comprises the amino acid sequence set forth in SEQ ID NO: 20; and
the second antigen-binding functional region that specifically binds to 4-1BB comprises
(A) a heavy chain variable region, comprising
   (a) HCDR1, which comprises the amino acid sequence set forth in SEQ ID NO: 21,
   (b) HCDR2, which comprises the amino acid sequence set forth in SEQ ID NO: 22, and
   (c) HCDR3, which comprises the amino acid sequence set forth in SEQ ID NO: 23; and
(B) a light chain variable region, comprising
   (a) LCDR1, which comprises the amino acid sequence set forth in SEQ ID NO: 24,
   (b) LCDR2, which comprises the amino acid sequence set forth in SEQ ID NO: 25, and
   (c) LCDR3, which comprises the amino acid sequence set forth in SEQ ID NO: 26.

The six CDRs of the first antigen-binding functional region that specifically binds to PD-L1 as set forth in SEQ ID Nos. 15-20, and the six CDRs of the second antigen-binding functional region that specifically binds to 4-1BB as set forth in SEQ ID Nos. 21-26 are the CDRs of the heavy and light chain variable regions of the monoclonal antibodies obtained by the inventors using hybridoma technology with human PD-L1 and 4-1BB as the antigens. Said monoclonal antibodies are different from the PD-L1 antibodies and 4-1BB antibodies known in the prior art and have higher biological activities, such as higher binding specificity, anti-tumor activity and so on.

The six CDR sequences of the first antigen-binding functional region that specifically binds to PD-L1 may have at least 70%, such as at least 75%, 80%, 85%, 90%, 95% or higher identity to the sequences set forth in SEQ ID Nos. 15-20, respectively, and retain the biological activities of the corresponding parent sequences; or may contain one or more, such as 1, 2, 3 or more amino acid deletions, substitutions and/or additions compared to the sequences set forth in SEQ ID Nos. 15-20, respectively, and retain the biological activities of the corresponding parent sequences.

The six CDR sequences of the second antigen-binding functional region that specifically binds to 4-1BB may have at least 70%, such as at least 75%, 80%, 85%, 90%, 95% or higher identity to the sequences set forth in SEQ ID Nos. 21-26, respectively, and retain the biological activities of the corresponding parent sequences; or may contain one or more, such as 1, 2, 3 or more amino acid deletions, substitutions and/or additions compared to the sequences set forth in SEQ ID Nos. 21-26, respectively, and retain the biological activities of the corresponding parent sequences.

In some embodiments, the first antigen-binding functional region of the bispecific antibody that specifically binds to PD-L1 comprises a heavy chain variable region and a light chain variable region, wherein the heavy chain variable region comprises the amino acid sequence set forth in SEQ ID NO: 6; or has at least 70%, such as at least 75%, 80%, 85%, 90%, 95%, 96%, 97%, 98%, 99% or higher identity to the sequence set forth in SEQ ID NO: 6 and retains the biological activities of the corresponding parent sequence; or contains one or more, such as 1, 2, 3, 4, 5, 6, 7, 8, 9, 10, 15, 20 or more amino acid deletions, substitutions and/or additions compared to the sequence set forth in SEQ ID NO: 6 and retains the biological activities of the corresponding parent sequence; and the light chain variable region comprises the amino acid sequence set forth in SEQ ID NO: 2; or has at least 70%, such as at least 75%, 80%, 85%, 90%, 95%, 96%, 97%, 98%, 99% or higher identity to the sequence set forth in SEQ ID NO: 2 and retains the biological activities of the corresponding parent sequence; or contains one or more, such as 1, 2, 3, 4, 5, 6, 7, 8, 9, 10, 15, 20 or more amino acid deletions, substitutions and/or additions compared to the sequence set forth in SEQ ID NO: 2 and retains the biological activities of the corresponding parent sequence.

In some embodiments, the second antigen-binding functional region of the bispecific antibody that specifically binds to 4-1BB comprises a heavy chain variable region and a light chain variable region, wherein the heavy chain variable region comprises the amino acid sequence set forth in SEQ ID NO: 12; or has at least 70%, such as at least 75%, 80%, 85%, 90%, 95%, 96%, 97%, 98%, 99% or higher identity to the sequence set forth in SEQ ID NO: 12 and retains the biological activities of the corresponding parent sequence; or contains one or more, such as 1, 2, 3, 4, 5, 6, 7, 8, 9, 10, 15, 20 or more amino acid deletions, substitutions and/or additions compared to the sequence set forth in SEQ ID NO: 12 and retains the biological activities of the corresponding parent sequence; and the light chain variable region comprises the amino acid sequence set forth in SEQ ID NO: 10; or has at least 70%, such as at least 75%, 80%, 85%, 90%, 95%, 96%, 97%, 98%, 99% or higher identity to the sequence set forth in SEQ ID NO: 10 and retains the biological activities of the corresponding parent sequence; or contains one or more, such as 1, 2, 3, 4, 5, 6, 7, 8, 9, 10, 15, 20 or more amino acid deletions, substitutions and/or additions compared to the sequence set forth in SEQ ID NO: 10 and retains the biological activities of the corresponding parent sequence.

In some embodiments, the first antigen-binding functional region of the bispecific antibody that specifically binds to PD-L1 comprises a heavy chain variable region and a light chain variable region, wherein the heavy chain variable region comprises the amino acid sequence set forth in SEQ ID NO: 6, and the light chain variable region comprises the amino acid sequence set forth in SEQ ID NO: 2; and the second antigen-binding functional region that specifically binds to 4-1BB comprises a heavy chain variable region and a light chain variable region, wherein the heavy chain variable region comprises the amino acid sequence set forth in SEQ ID NO: 12, and the light chain variable region comprises the amino acid sequence set forth in SEQ ID NO: 10.

Those skilled in the art would know that the combinations of the amino acid sequences should follow the biological laws, i.e., light and heavy chains or the variable regions, the antigen-binding fragments thereof, such as SEQ ID NO: 6 and SEQ ID NO: 2, and SEQ ID NO: 12 and SEQ ID NO: 10, should match each other.

In some embodiments, the first antigen-binding functional region and the second antigen-binding functional region of the bispecific antibody are selected from the group consisting of Fab fragments, scFv fragments, and variable domain fragments Fv.

In some embodiments, the first antigen-binding functional region and the second antigen-binding functional region of the bispecific antibody are both Fab fragments.

In some embodiments, the Fab fragment of the bispecific antibody comprises a different first heavy chain variable region and a different second heavy chain variable region, as well as a different first light chain variable region and a different second light chain variable region.

In some embodiments, one of the first antigen-binding functional region and the second antigen-binding functional region of the bispecific antibody is a Fab fragment, and the other is a scFv.

In some embodiments, the bispecific antibody comprises a first Fc chain and a second Fc chain, and a first antigen-binding functional region capable of specifically binding to PD-L1 and a second antigen-binding functional region capable of specifically binding to 4-1BB,
wherein the first Fc chain and the second Fc chain are both immunoglobulin G Fc fragments containing amino acid substitutions, and the first Fc chain and the second Fc chain together constitute a heterodimer that can bind to the Fc receptor;
wherein the first Fc chain and the second Fc chain are linked to the first antigen-binding functional region and the second antigen-binding functional region, respectively, via a covalent bond or a linker; and
wherein either the first Fc chain or the second Fc chain comprises amino acid substitutions at positions 366 and 399, and the other comprises amino acid substitutions at positions 351, 407 and 409, wherein the amino acid positions are numbered according to the Kabat EU index numbering system.

In some embodiments, the amino acid substitutions of the first Fc chain and the second Fc chain of the bispecific antibody are as follows,
a) L351G, L351Y, L351V, L351P, L351D, L351E, L351K or L351W;
b) T366L, T366P, T366W or T366V;
c) D399C, D399N, D399I, D399G, D399R, D399T or D399A;
d) Y407L, Y407A, Y407P, Y407F, Y407T or Y407H; and
e) K409C, K409P, K409S, K409F, K409V, K409Q or K409R.

In some embodiments, the amino acid substitutions of the bispecific antibody include:
a) substitutions T366L and D399R on either the first Fc chain or the second Fc chain, and substitutions L351E, Y407L and K409V on the other;
b) substitutions T366L and D399C on either the first Fc chain or the second Fc chain, and substitutions L351G, Y407L and K409C on the other;
c) substitutions T366L and D399C on either the first Fc chain or the second Fc chain, and substitutions L351Y, Y407A and K409P on the other;
d) substitutions T366P and D399N on either the first Fc chain or the second Fc chain, and substitutions L351V, Y407P and K409S on the other;
e) substitutions T366W and D399G on either the first Fc chain or the second Fc chain, and substitutions L351D, Y407P and K409S on the other;
f) substitutions T366P and D399I on either the first Fc chain or the second Fc chai, and substitutions L351P, Y407F and K409F on the other;
g) substitutions T366V and D399T on either the first Fc chain or the second Fc chain, and substitutions L351K, Y407T and K409Q on the other;
h) substitutions T366L and D399A on either the first Fc chain or the second Fc chain, and substitutions L351W, Y407H and K409R on the other.

In some embodiments, the amino acid substitutions of the bispecific antibody include:
a) substitutions T366L and K409V on either the first Fc chain or the second Fc chain, and substitutions L351E, Y407L and D399R on the other;
b) substitutions T366L and K409C on either the first Fc chain or the second Fc chain, and substitutions L351G, Y407L and D399C on the other;
c) substitutions T366L and K409P on either the first Fc chain or the second Fc chain, and substitutions L351Y, Y407A and D399C on the other;
d) substitutions T366P and K409S on either the first Fc chain or the second Fc chain, and substitutions L351V, Y407P and D399N on the other;
e) substitutions T366W and K409S on either the first Fc chain or the second Fc chain, and substitutions L351D, Y407P and D399G on the other;
f) substitutions T366P and K409F on either the first Fc chain or the second Fc chain, and substitutions L351P, Y407F and D399I on the other;
g) substitutions T366V and K409Q on either the first Fc chain or the second Fc chain, and substitutions L351K, Y407T and D399T on the other;
h) substitutions T366L and K409R on either the first Fc chain or the second Fc chain, and substitutions L351W, Y407H and D399A on the other.

In some embodiments, the substitutions on either the first Fc chain or the second Fc chain of the bispecific antibody are T366L and D399R, and the substitutions on the other are L351E, Y407L and K409V.

In some embodiments, the weight ratios of the homodimers formed by the first Fc chain and the first antigen-binding functional region covalently linked thereto and by the second Fc chain and the second antigen -binding functional region covalently linked thereto of said bispecific antibody are less than 50%, such as less than 45%, 40%, 35%, 30%, 25%, 20% or less of the total amount of all the polypeptide chains, in a reducing agent-containing solution in which no other polypeptides present except for said Fc chains and the antigen-binding functional regions hereinabove.

In some embodiments, the bispecific antibody comprises a first heavy chain/first light chain pair that specifically binds to PD-L1, wherein the first heavy chain has an heavy chain variable region comprising the amino acid sequence set forth in SEQ ID NO: 6, and a heavy chain constant region comprising the amino acid sequence set forth in SEQ ID NO: 8; the first light chain has a light chain variable region comprising the amino acid sequence set forth in SEQ ID NO: 2, and a light chain constant region comprising the amino acid sequence set forth in SEQ ID NO: 4.

In some embodiments, the bispecific antibody comprises a second heavy chain/second light chain pair that specifically binds to 4-1BB, wherein the second heavy chain has an heavy chain variable region comprising the amino acid sequence set forth in SEQ ID NO: 12, and a heavy chain constant region comprising the amino acid sequence set forth in SEQ ID NO: 14; the second light chain has a light chain variable region comprising the amino acid sequence set forth in SEQ ID NO: 10, and a light chain constant region comprising the amino acid sequence set forth in SEQ ID NO: 4.

A second aspect of the present invention relates to an isolated polynucleotide encoding the bispecific antibody in a heterodimeric form as described above.

In some embodiments, the nucleotide sequences encoding the amino acid sequences of the first antigen-binding functional region comprise the nucleotide sequences set forth in SEQ ID NOs: 1 and 5.

In some embodiments, the nucleotide sequences encoding the amino acid sequences of the second antigen-binding functional region comprise the nucleotide sequences set forth in SEQ ID NOs: 11 and 9.

In some embodiments, the nucleotide sequence encoding the amino acid sequences of the first light chain and the second light chain both comprises the nucleotide sequence set forth in SEQ ID NO: 3.

In some embodiments, the nucleotide sequence encoding the amino acid sequence of either the first heavy chain or the second heavy chain comprises the nucleotide sequence set forth in SEQ ID NO: 7, and the nucleotide sequence encoding the amino acid sequence of the other comprises the nucleotide sequence set forth in SEQ ID NO: 13.

A third aspect of the present invention relates to a recombinant expression vector comprising the isolated polynucleotide as described above.

In some embodiments, the expression vector is the plasmid vector XOGC modified from the pCDNA vector.

A fourth aspect of the present invention relates to a host cell comprising the isolated polynucleotide as described above or the recombinant expression vector as described above.

In some embodiments, the host cell is selected from the group consisting of human embryonic kidney cells HEK293, or HEK293T, HEK293E, HEK293F that are modified from HEK293 cells; hamster ovary cells CHO, or CHO-S, CHO-dhfr⁻, CHO/DG44, ExpiCHO that are modified from CHO cells; *Escherichia coli,* or *E. coli* BL21, BL21(DE3), Rosetta, Origami that are modified from *E. coli*; Yeast, or *Pichia, Saccharomyces cerevisiae, Kluyveromyces lactis, Hansenula polymorpha* that are modified from Yeast; insect cells, or High5, SF9 cells that are modified from insect cells; plant cells; mammalian mammary gland cells; somatic cells.

A fifth aspect of the present invention relates to a composition comprising the bispecific antibody as described above or the isolated polynucleotide as described above or the recombinant expression vector as described above or the host cell as described above, and a pharmaceutically acceptable carrier. In further embodiments, the composition further comprises a second therapeutic agent, preferably, the second therapeutic agent and the bispecific antibody, the isolated polynucleotide, the recombinant expression vector or the host cell are located in different parts of the composition, and preferably, the second therapeutic agent is an anti-PD-1 antibody and/or a STING agonist.

A sixth aspect of the present invention relates to a method of producing the bispecific antibody as described above, including the steps of:
1) Expressing the isolated polynucleotide as described above or the recombinant expression vector as described above in host cells, respectively;
2) Reducing the proteins respectively expressed in the host cells; and
3) Mixing the reduced proteins, and then oxidizing the mixture.

In some embodiments, the host cell is selected from the group consisting of human embryonic kidney cells HEK293, or HEK293T, HEK293E and HEK293F that are modified from HEK293 cells; hamster ovary cells CHO, or CHO-S, CHO-dhfr-, CHO/DG44, ExpiCHO that are modified from CHO cells; *Escherichia coli,* or *E. coli* BL21, BL21(DE3), Rosetta, Origami that are modified from *E. coli*; Yeast, or *Pichia, Saccharomyces cerevisiae, Kluyveromyces lactis, Hansenula polymorpha* that are modified from Yeast; insect cells, or High5, SF9 cells that are modified from insect cells; plant cells; mammalian mammary gland cells, somatic cells.

In some embodiments, the reduction step includes 1) performing the reduction reaction in the presence of a reducing agent selected from the group consisting of 2-mercaptoethylamine, dithiothreitol, tris(2-carboxyethyl) phosphine or other chemical derivatives thereof; 2) Removing the reducing agent. In some embodiments, the reducing agent is dithiothreitol at a concentration of 0.1 mM or higher, such as 0.1 mM, 0.2 mM, 0.3 mM, 0.4 mM, 0.5 mM or higher, and the reaction is performed at 4°C for at least 3 hours, such as 3.5 hours, 4 hours, 4.5 hours, 5 hours, 5.5 hours, 6 hours or longer.

In some embodiments, the oxidation step is conducted in the air or in the presence of an oxidizing agent selected from the group consisting of L-dehydroascorbic acid or chemical derivatives thereof. In some embodiments, the oxidizing agent is L-dehydroascorbic acid at a concentration of 0.5 mM or higher, such as 0.6 mM, 0.7 mM, 0.8 mM, 0.9 mM, 1.0 mM, 1.2 mM, 1.5 mM or higher, and the reaction is performed at 4°C for at least 5 hours, such as 5 hours, 6 hours, 7 hours, 8 hours, 9 hours, or longer.

In some embodiments, the method further includes a step of separation and purification. In some embodiments, the separation and purification include cation resin exchange, anion resin exchange, reverse phase chromatography, affinity chromatography, size exclusion chromatography, and combinations thereof.

A seventh aspect of the present invention relates to use of the bispecific antibody as described above and/or the isolated polynucleotide as described above and/or the recombinant expression vector as described above and/or the host cell as described above and/or the composition as described above, in the manufacture of a medicament for preventing and/or treating diseases in a subject.

An eighth aspect of the present invention relates to the bispecific antibody as described above and/or the isolated polynucleotide as described above and/or the recombinant expression vector as described above and/or the host cell as described above and/or the composition as described above, for use as a medicament for preventing and/or treating diseases in a subject.

A ninth aspect of the present invention relates to a method of preventing and/or treating diseases, including administering to a subject in need thereof the bispecific antibody as described above and/or the isolated bispecific antibody as described above and/or the recombinant expression vector as described above and/or the host cell as described above and/or the composition as described above.

In some embodiments, the subject is a mammal, preferably a human subject.

In some embodiments, the disease is selected from the group consisting of the following tumors: leukemia, lymphoma, myeloma, brain tumor, head and neck squamous cell cancer, non-small cell lung cancer, nasopharyngeal cancer, esophageal cancer, gastric cancer, pancreatic cancer, gallbladder cancer, liver cancer, colorectal cancer, breast cancer, ovarian cancer, cervical cancer, endometrial cancer, uterine sarcoma, prostate cancer, bladder cancer, renal cell cancer, melanoma, small cell lung cancer and bone cancer.

In other words, the present invention designs a novel anti-PD-L1/anti-4-1BB bispecific antibody in the form of a natural antibody structure-like heterodimer with the characteristics of natural IgGs and no heavy and light chain mismatches, and thus it is a highly stable anti-PD-L1/anti-4-1BB bispecific antibody in a heterodimeric form. The bispecific antibody prepared in the invention is able to bind to two target molecules, PD-L1 and 4-1BB simultaneously, and to exert better efficacy with fewer side effects than a monotherapeutic agent when applied to treat complicated diseases. Moreover, compared to a combination therapy of multiple medicaments, the bispecific antibody, as a single therapeutic molecule, not only facilitates the use by patients and medical workers, but also simplifies the complex development processes for new drugs.

### Brief description of the figures

Fig. 1 illustrates a chromatogram of elution peaks of the anti-PD-L1 expression product.
Fig. 2 illustrates a chromatogram of elution peaks of the anti-4-1BB expression product.
Fig. 3 illustrates a structure of the anti-PD-L1/anti-4-1BB bispecific antibody molecule.
Fig. 4 illustrates a molecular structure of a half-antibody comprising one heavy chain and one light chain.
Fig. 5 illustrates results of SEC-HPLC analysis of the half-antibody molecule containing one heavy chain and one light chain, wherein panels A and B illustrate the results of the anti-PD-L1 half antibody molecule and the anti-4-1BB half antibody molecule, respectively.
Fig. 6 illustrates results of SEC-HPLC analysis of the anti-PD-L1/anti-4-1BB bispecific antibody molecule.
Fig. 7 illustrates results of CE analysis of the anti-PD-L1/anti-4-1BB bispecific antibody molecule.
Fig. 8 Panel A illustrates the affinity of the anti-PD-L1/anti-4-1BB bispecific antibody to PD-L1, panel B illustrates the affinity of the anti-PD-L1/anti-4-1BB bispecific antibody to 4-1BB, and panel C illustrates the affinity of anti-PD-L1/anti-4-1BB bispecific antibody to 4-1BB.
Fig. 9 Panel A illustrates the activity of the anti-PD-L1/anti-4-1BB bispecific antibody blocking the binding to PD-L1/PD-1, and panel B illustrates the activity of the anti-PD-L1/anti-4-1BB bispecific antibody blocking the binding to PD-L1/CD80.
Fig. 10 illustrates the T cell regulatory activity of the anti-PD-L1/anti-4-1BB bispecific antibody.
Fig. 11 illustrates the 4-1BB agonistic activity mediated by the anti-PD-L1/anti-4-1BB bispecific antibody.
Fig. 12 illustrates the anti-tumor efficacy of the anti-PD-L1/anti-4-1BB bispecific antibody in the gene knock-in mouse homologous tumor model.
Fig. 13 illustrates the tumor volume changes of BALB/c-hPD1/hPDL1/hCD137 mice.
Fig. 14 illustrates the tumor volume data of h4-1BB/hPD-1 mice.

### Detailed description of the embodiments

### Definitions

As used herein, the term "antibody" is used in its broadest sense and refers to a protein containing antigen-binding site(s), including natural antibodies and artificial antibodies with various structures, including but not limited to intact antibodies and antigen-binding fragments of the antibodies.

As used herein, the term "bispecific antibody" comprises antigen binding domains specifically binding to epitopes on two different biological molecules. Unless otherwise specified, the order of the antigens bound by the bispecific antibody is arbitrary. That is, in some embodiments, the terms "anti-PD-L1/4-1BB bispecific antibody" and "anti-4-1BB/PD-L1 bispecific antibody" are used interchangeably. In some embodiments, the bispecific antibody comprises two half-antibodies, wherein each half-antibody comprises a single heavy chain variable region and optionally at least part of the heavy chain constant region, as well as a single light chain variable region and optionally at least part of the light chain constant region. In some embodiments, the bispecific antibody comprises two half-antibodies, wherein each half-antibody comprises a single heavy chain variable region and a single light chain variable region, and comprises no more than one single heavy chain variable region and no more than one single light chain variable region. In some embodiments, the bispecific antibody comprises two half-antibodies, wherein each half-antibody comprises a single heavy chain variable region and a single light chain variable region, and wherein the first half-antibody binds to the first antigen and not to the second antigen, and the second half-antibody binds to the second antigen and not to the first antigen.

The term "complementarity determining region" or "CDR region" or "CDR" (used interchangeably with hypervariable region "HVR" herein) refers to a region in an antibody variable domain that is hypervariable in sequence and forms a structure-defined loop ("a hypervariable loop") and/or contains antigen contact residues ("antigen contact points"). CDRs are mainly responsible for binding to an antigen epitope. In the present invention, the three CDRs of the heavy chain are referred to as HCDR1, HCDR2 and HCDR3, and the three CDRs of the light chain are referred to as LCDR1, LCDR2, and LCDR3.

It should be noticed that the boundaries of the CDRs of the variable regions of the same antibody obtained based on different numbering systems may be different. That is, the CDR sequences of the same antibody variable region defined under different numbering systems can be different. Therefore, for an antibody limited by specific CDR sequences defined according to the present invention, the scope of the antibody also encompasses antibodies whose variable region sequences comprises said specific CDR sequences but with different indicated CDR boundary from the specific CDR boundary defined in the present invention due to the application of different schemes (for example, different numbering system rules or combinations thereof).

Covalent linkage means that two Fc chains, or any one of the Fc chains and an antigen-binding functional region linked thereto in a heterodimeric bispecific antibody are linked with each other via a covalent bond to form a single molecule. Among them, the Fc chains comprise a first antigen-binding functional region and a second antigen-binding functional region linked via one or more covalent bonds (e.g., a disulfide bond); the first and second Fc chains are linked to an antigen-binding functional region via a covalent linkage (e.g., an imine or amide linkage), respectively.

An antigen-binding functional region is a region which can specifically interact with a target molecule, such as an antigen, and whose action is highly selective, and a sequence that recognizes one target molecule is generally unable to recognize other molecular sequences. A representative antigen-binding functional region includes an antibody variable region, a structural variant of an antibody variable region, a receptor binding domain, a ligand binding domain, or an enzyme binding domain.

One or more disulfide bond inter-chain linkages refer to the formation of a heterodimeric fragment by linkages between the first Fc chain and the second Fc chain via one or more disulfide bonds. In the present invention, one or more disulfide bonds may be formed when the first Fc chain and the second Fc chain or the first Fc chain and the second Fc chain and the antigen-binding functional regions linked thereto are synthesized in a same cell, or may be formed by *in vitro* reduction-oxidation after the first Fc chain and the second Fc chain or the first Fc chain and the second Fc chain and the antigen-binding functional regions linked thereto are synthesized in different cells, respectively.

The first Fc chain and the second Fc chain refer to forming a binding fragment through a covalent linkage, wherein the covalent linkage comprises a disulfide bond, and each chain comprises at least one portion of the heavy chain constant region of an immunoglobulin; and the first Fc chain and the second Fc chain are different from each other in their amino acid sequences, and comprise at least one different amino acid. As to the first and second Fc chains of the present invention, a strong, mutual, repulsive force exists between identical chains, and attractive force exists between different chains. Accordingly, the first and second Fc chains, or the first and second Fc chains and the antigen-binding functional regions linked thereto have a tendency to undergo heterodimeric formation, when co-expressed in a cell. When the first and second Fc chains, or the first and second Fc chains and the antigen binding domain linked thereto are expressed in two host cells, respectively, the first Fc chains, or the first Fc chain and the antigen binding domain linked thereto have no tendency to undergo homodimeric formation, and the second Fc chains, or the second Fc chain and the antigen binding domain linked thereto have no tendency to undergo homodimeric formation. In the present invention, when the first and second Fc chains, or the first and second Fc chains and the antigen-binding functional regions linked thereto are expressed in two host cells, respectively, and a reducing agent is present, a percentage of homodimers is less than 50%, that is, a percentage of monomers (one Fc chain or one Fc chain and one antigen-binding functional region linked thereto) is above 50%.

An immunoglobulin has a symmetrical structure having four polypeptide chains, wherein two chains are identical heavy chains which are relatively long and have a relatively high molecular weight, each including 450-550 amino acid residues and having a relative molecular weight of 55000-70000 Da; and the other two chains are identical light chains (L chains) which are relatively short and have a relatively low molecular weight, including about 210 amino acid residues and having a relative molecular weight of about 24000 Da. Sequences of about 110 amino acids in length near the N-termini of the heavy and light chains of an immunoglobulin are highly variable, and are called variable regions (V regions), while the rest amino acid segments near the C-termini thereof are relatively stable and called constant regions (C regions). Each heavy chain of the antibody consists of a heavy chain variable region (referred to as VH herein) and a heavy chain constant region (referred to as CH herein), and each light chain consists of a light chain variable region (referred to as VL herein) and a light chain constant region (referred to as CL herein). The variable region in the heavy chain occupies approximately 1/4 of the length of the heavy chain, and the constant region occupies approximately 3/4 of the length of the heavy chain. The known five types of Igs are IgG (γ), IgA (α), IgD (δ), IgM (µ) and IgE *(*ε). Among them, the first three types of Igs have three constant regions in the H chain, namely, CH1, CH2 and CH3. The latter two types of Igs (IgM and IgE) have one VH region and four constant regions in the H chain, namely CH1 to CH4. The constant regions are not only the framework of an immunoglobulin molecule but also one of the regions activating immune responses. Although embodiments of the invention relate to IgG, those skilled in the art would have recognized that the class of the antibodies of the invention can be switched by known methods, if desired. For example, an antibody of the invention that is initially IgM can be class-switched to an IgG antibody of the invention. In addition, class switching techniques can be used to convert one IgG subclass to another, such as from IgG1 to IgG2. Thus, the effector functions of the antibodies of the invention can be switched by isotypes to, for example, IgG1, IgG2, IgG3, IgG4, IgD, IgA, IgE or IgM antibodies for various therapeutic uses. In one embodiment, the antibody of the invention is an IgG1 antibody, such as IgG1, κ,.

A part of the constant region of the present disclosure includes at least an interaction region of the first Fc chain and the second Fc chain, and in the case of IgG, this region is part of amino acids located in the CH3 domain and includes at least GLN 347, TYR 349, THR 350, LEU 351, SER 354, ARG 355, ASP 356, GLU 357, LYS 360, SER 364, THR 366, LEU 368, LYS 370, ASN 390, LYS 392, THR 394, PRO 395, VAL 397, ASP 399, SER 400, PHE 405, TYR 407, LYS 409, and LYS 439.

The first Fc chain and the second Fc chain each are linked to an antigen-binding functional region via a covalent bond or a linker, meaning that the first Fc chain and the second Fc chain each are linked to an antigen-binding fragment of an antibody, or a single chain antibody capable of recognizing an antigen, or other antibody fragment variant capable of recognizing an antigen, or a receptor capable of recognizing a ligand, or a ligand capable of recognizing a receptor via a covalent bond or a linker. A covalent bond is one of chemical bonds, in which two or more atoms together use their outer electrons, to reach electronic saturation state in an ideal situation, thus forming a relatively stable chemical structure named a covalent bond. In other words, a covalent bond is an interaction between atoms by sharing their electrons. The atoms of the same element or different elements can both bind to each other via covalent bonds. The covalent bonds between the first Fc chain and the second Fc chain of the invention include, but are not limited to, an amide bond formed by carboxyl dehydration reaction between an amino group of an amino acid molecule and a carboxyl group of another amino acid molecule, or an amide bond or imine bond formed between an aldehyde group of ethylene glycol or polyethylene glycol or other compound or a polymer thereof and an amino group of an amino acid molecule; wherein the linker is one stretch of amino acid sequence or one compound or one multimer of one compound capable of linking two polypeptide chains via covalent bonds, wherein the one stretch of amino acid sequence includes, but is not limited to, a small peptide, such as GGGGSGGGGSGGGGS, which links together the first Fc chain or the second Fc chain, and a single-chain antibody capable of recognizing an antigen, or other antibody fragment structural variant capable of recognizing an antigen via amide bonds.

The first Fc chain and the second Fc chain have a tendency to undergo heterodimeric formation, rather than a tendency to undergo homodimeric formation, which means that as in the first Fc chain and the second Fc chain, a repulsive force exists between identical polypeptide chains and an attractive force exists between different polypeptide chains, and therefore, the first Fc chain and the second Fc chain, or the first and second Fc chains and the antigen-binding functional regions linked thereto have a tendency to undergo heterodimeric formation, when co-expressed in a cell. When the first Fc chain and the second Fc chain, or the first and second Fc chains and the antigen-binding functional regions linked thereto are expressed in two host cells, respectively, the first Fc chains, or the first Fc chain and the antigen-binding functional region linked thereto have no tendency to undergo homodimeric formation, and the second Fc chains, or the second Fc chain and the antigen-binding functional region linked thereto also have no tendency to undergo homodimeric formation.

The Kabat EU index numbering system refers to a method used by Kabat to assign a number for each amino acid of an antibody sequence, and this method for assigning a number for each residue has become a standard method in the art. The Kabat protocol can be applied to other antibodies that are not in its research. Based on conserved amino acids, the target antibody is aligned with one of the consensus sequences identified by Kabat. In the context of present invention, unless otherwise specified, amino acid positions of an antibody are all numbered according to the Kabat EU index numbering system.

The Fc domain refers to a fragment crystallizable (Fc), corresponds to CH2 and CH3 domains of an Ig, and is a site where an Ig interacts with an effector molecule or a cell.

IgG is an abbreviation of immunoglobulin G (IgG), and is the main type of antibodies in serum. Human IgG has four subclasses, namely IgG1, IgG2, IgG3 and IgG4, based on antigenic differences in r-chains in the IgG molecules.

A half-antibody molecule refers to a structure formed by one heavy chain and one light chain of an antibody, wherein the heavy chain and the light chain may be linked via or not via a covalent bond, and is a monovalent antibody structure recognizing an antigen.

A Fab fragment is a molecule-recognizing sequence, i.e. a fragment of antigen binding (Fab), and corresponds to two arms of an antibody molecule, consisting of a complete light chain and VH and CH1 domains of a heavy chain. scFv, a molecule- recognizing sequence, is a structural variant of an antibody fragment obtained by genetic engineering modification of a light chain variable region and a heavy chain variable region of an antibody. An extracellular region of a membrane receptor is a molecule-recognizing sequence, and the membrane receptor generally comprises an extracellular region located outside the cell that recognizes and binds to the corresponding antigen or ligand, a transmembrane region that anchors the receptor onto the cell surface, and an intracellular region in a cell that has kinase activity or transfers a signal. A ligand of the cell membrane receptor refers to a protein, a small peptide or a compound that may be recognized and bound by the extracellular region of a membrane receptor. Cytokines are low-molecular weight soluble proteins that are produced due to the induction of various types of cells by immunogens, mitogens or other stimulants, and have various functions such as regulations of innate immunity and adaptive immunity, hematopoiesis, cell growth, APSC pluripotent cell, and damage tissue repair, etc. Cytokines may be classified into interleukins, interferons, tumor necrosis factor superfamilies, colony stimulating factors, chemokines, growth factors, etc. A protein expression tag means an amino acid sequence added to the N-terminus or C-terminus of a target protein, and may be a small peptide or a long amino acid sequence. Addition of a tag may be advantageous for correct folding of proteins, isolation and purification of proteins, and decreasing intracellular degradation of proteins. Tags frequently used may include, but not limited to HA, SUMO, His, GST, GFP and Flag.

There is no restriction on the antibodies used for the bispecific antibody in a heterodimeric form of the present invention. Preferably, all the antibodies known in the prior art that can be used for treating and/or preventing diseases may be used in the present invention.

The bispecific antibody in a heterodimeric form of the present invention may have one or more substitutions, deletions, additions and/or insertions. For example, certain amino acids can substitute other amino acids in the protein structure without significant loss of the ability to bind to other polypeptides (such as antigens) or cells. Since the binding ability and protein properties determine the bioactivities and functions of a protein, substitutions of certain amino acid sequences can be made in the protein sequence without significant loss of their biological efficacy or activities.

In many cases, a polypeptide variant contains one or more conservative substitutions. The "conservative substitution" means that in which an amino acid in the polypeptide is replaced by another amino acid with similar properties, such that the skilled in the peptide chemistry field would anticipate that the secondary structure and hydrophilic property of the polypeptide are substantially unchanged.

Amino acid substitutions are generally based on the relative similarity of the amino acid side chain substituents, such as their hydrophobicity, hydrophilicity, charges, size, etc. Exemplary substitutions that take various aforementioned characteristics into account are well known to those skilled in the art and include: arginine and lysine; glutamic acid and aspartic acid; serine and threonine; glutamine and asparagine; and valine, leucine and isoleucine.

As used herein, the term "identity" has the meaning generally known in the art. Those skilled in the art are also familiar with the rules and criteria for determining the identity between different sequences, and "identity" refers to the percentage of residues in a variant polynucleotide or polypeptide sequence that are identical to that of a non-variant sequence after aligning the sequences and introducing gaps (if necessary, in order to obtain the maximum percent homology). In the present invention, under the condition that the identity restriction is met, the obtained variant sequence also needs to have the biological activities possessed by the parent sequence. The skilled in the art would have well known the methods and means for screening variant sequences according to the above-described activities. Such variant sequences could be readily obtained by those skilled in the art in the light of the disclosure of the present application. In a specific embodiment, the polynucleotide and polypeptide variants have at least about 70%, at least about 75%, at least about 80%, at least about 90%, at least about 95%, at least about 98%, or at least about 99%, or at least about 99.1%, 99.2%, 99.3%, 99.4%, 99.5%, 99.6%, 99.7%, 99.8%, or 99.9% polynucleotide or polypeptide identity with the polynucleotide and polypeptide described herein. Due to the redundancy of the genetic codons, there will be variants of these sequences encoding the same amino acid sequence.

In another embodiment of the present invention, a polynucleotide composition capable of hybridizing with the polynucleotide sequence or the fragment or the complementary sequence thereof provided in the present invention under moderately to highly stringent conditions is provided. The hybridization techniques are well known in the art of molecular biology. For the purposes of illustration, suitable moderately stringent condition for testing the hybridization of the polynucleotide of the present invention with other polynucleotides include pre-washing with a solution of 5×SSC, 0.5% SDS, and 1.0 mM EDTA (pH 8.0); hybridizing at 50-60°C, 5×SSC, overnight; and then washing twice with 2×, 0.5× and 0.2× SSC containing 0.1% SDS at 65°C for 20 minutes. Those skilled in the art would recognize that the stringency of hybridization may be easily manipulated, for example, by changing the salt content of the hybridization solution and/or the temperature during hybridization. For example, in another embodiment, suitable highly stringent hybridization conditions include the conditions described above, except that the hybridization temperature is increased, for example, to 60-65°C or 65-70°C.

The host cell of the present invention may be any cell used for exogenous gene expression, including but not limited to *Escherichia coli,* yeast, insect cells, plant cells, and mammalian cells.

The vector of the present invention includes a vector that may replicate in any type of cells or organisms, including, for example, plasmids, bacteriophages, cosmids, and mini-chromosomes. In some embodiments, the vector comprising the polynucleotide of the present invention is a vector suitable for propagation or replication of the polynucleotide, or a vector suitable for expressing the polypeptide of the present invention. Such vectors are known in the art and are commercially available.

The "vector" includes shuttle vectors and expression vectors. In general, a plasmid construct also includes an origin of replication (such as the ColE1 origin of replication) and a selectable marker (such as ampicillin or tetracycline resistance) for plasmid replication and selection in bacteria, respectively. The "expression vector" refers to a vector containing the control sequences or regulatory elements required for expressing the antibody of the present invention, including antibody fragments, in bacteria or eukaryotic cells.

The vector of the present invention may be any vector used for the exogenous gene expression, including but not limited to plasmid vectors, which contains at least an origin of replication, a promoter, a target gene, a multiple cloning site, and a gene of the selectable marker, and preferably, the vector of the present invention includes but is not limited to modified plasmid vector obtained based on pCDNA, such as XOGC vector.

The composition of present invention further comprises a second therapeutic agent, preferably, the second therapeutic agent and the bispecific antibody, the isolated polynucleotide, the recombinant expression vector or the host cell are located in different parts of the composition, and preferably, the second therapeutic agent is an anti-PD-1 antibody and/or a STING agonist.

The second therapeutic agent comprised in the composition of present invention has no special limitation, provided that it is physiologically compatible with the bispecific antibody, the isolated polynucleotide, the recombinant expression vector or the host cell of present invention. The term "physiologically compatible" means that there is no excessive stimulation, toxicity and other adverse reactions when administered to the subject. In some embodiments, the second therapeutic agent and the bispecific antibody, the isolated polynucleotide, the recombinant expression vector or the host cell present in the composition as a mixture. In some embodiments, the second therapeutic agent and the bispecific antibody, the isolated polynucleotide, the recombinant expression vector or the host cell are located in different parts of the composition. The term "in different parts of the composition" means that the composition consists of parts separated from each other, and the separated parts may be located in different containers or in different compartments separated from each other in a same container, such as different vials, syringes, tubules. The parts separated from each other may be mixed together for co-administration prior to administration, or administered sequentially at certain intervals, such as an interval of 5 minutes, 10 minutes, 20 minutes, 30 minutes, 1 hour, 5 hours, 12 hours, 24 hours, 48 hours, 1 week, 1 month or longer. The second therapeutic agent may be a medicament for treating the same or different diseases as those to be treated by the bispecific antibody, the isolated polynucleotide, the recombinant expression vector or the host cell, for example, both for the treatment of leukemia, or one for the treatment of leukemia and the other for the treatment of colon cancer. In some embodiments, the second therapeutic agent includes an anti-inflammatory agent, such as IL-6 inhibitors, tumor necrosis factor α (TNF-α) inhibitors, interferon γ (IFN-γ) inhibitors, corticosteroids, antihistamines, antipyretics and / or antibiotics. In some embodiments, the second therapeutic agent is selected from a group consisting of: a second antibody, an immunotherapeutic agent, a targeted therapeutic agent or a chemotherapeutic agent.

In some embodiments, the second therapeutic agent is an antibody, and the target of the antibody selected from a group consisting of: CD47, CD70, CD200, CD154, CD223 (LAG-3), KIR (killer-cell immunoglobulin-like receptors), GITR, CD20, CD28, CD40, CD86, CD160, CD258, CD270, CD275, CD276, OX40L, B7-H4, GITRL, 4-1BBL, CD3 , CD25, CD48, CD66a, CD80, CD94, CD96, CD112, CD115, CD205, CD226, CD244, CD262, CD284, CD288, Leukemia Inhibitory Factor (LIF), TNFSF15, TDO2, IGF-1R, GD2, TMIGD2, RGMB, VISTA, BTNL2, Btn, TIGIT, Siglecs ((sialic acid binding Ig-like lectins, i.e. SIGLEC 15), VEGFR, ILT family, MICA, TGFβ, STING pathway (stimulator of interferon gene pathway), arginase, EGFRvIII, HHLA2, PD-1, PD-L1, PD-L2, CTLA-4, BTLA, indoleamine 2,3-dioxygenase (IDO1), TIM3, A2A adenosine receptor (ADO receptor), CD39, CD73, CD27, ICOS (CD278), CD137 (4 -1BB), OX40, TNFSF25, IL-10, Galectins, NKp30, NKp40, NKp44, NKp46, NKG2A, NKG2D, DNAM1, DAP10, CD16 (CD16a, CD16b, or both), CRTAM, CD27, PSGL1, CD96, CD100 (also known as SEMA4D), NKp80, CD244 (also known as SLAMF4 or 2B4), SLAMF6, SLAMF7, KIR2DS2, KIR2DS4, KIR3DS1, KIR2DS3, KIR2DS5, KIR2DS1, CD94, NKG2C, NKG2E or CD 160.

In some embodiments, the antibody is an anti-PD-1 antibody. In some embodiments, the anti-PD-1 antibody is nivolumab, pembrolizumab, cemiplimab, Camrelizumab, Toripalimab, sintilimab, tislelizumab, tislelizumab, Penpulimab or zimberelimab.

In some embodiments, the second therapeutic agent is an immunotherapeutic agent. Non-limiting examples of the immunotherapeutic agents include: antibodies, small molecule modulators, chimeric antigen receptor (CAR) T cells, NK cells, dendritic cells (DCs), adoptive cell transfer (ACT), immune checkpoint modulators, cytokines, cancer vaccines, adjuvants, oncolytic viruses, or combinations thereof. In some embodiments, the immunotherapy agents are STING agonists.

In some embodiments, the second therapeutic agent is a targeted therapeutic agent. The term "targeted therapeutic agent" refers to molecules that specifically target and inhibit one or more oncogenic signaling proteins (e.g., those involved in cell growth and survival). Non-limiting examples of such targeted therapeutic agents include: tyrosine-kinase inhibitors (e.g., imatinib (GLEEVEC^{®}), gefitinib (IRESSA^{®}), erlotinib (TARCEVA^{®}), sorafenib (NEXAVAR^{®}), sunitinib (SUTENT^{®}), dasatinib (SPRYCL^{®}), lapatinib (TYKERB^{®}), nilotinib (TASIGNA^{®}), bortezomib (VELCADE^{®}), tamoxifen (NOLVADEX^{®}), tofacitinib (XELJANZ^{®}), ALK inhibitors (e.g., crizotinib), Bcl-2 inhibitors (e.g., obatoclax, navitoclax, gossypol), PARP inhibitors (e.g., iniparib, olaparib), PI3K inhibitors (e.g., perifbsine), apatinib, AN-152, Braf inhibitors (e.g., trametinib, MEK162), CDK inhibitors (e.g., PD-0332991, LEE011), Hsp90 inhibitors, salinomycin, VAL-083)); small molecule drug conjugates (e.g., vintafolide); serine- threonine kinase inhibitors (e.g., temsirolimus (TORISEL^{®}), everolimus (AFINITOR^{®}), vemurafenib (ZELBORAF^{®}), trametinib (MEKINIST^{®}), dabrafenib (TAFINLAR^{®})); antibodies (e.g., anti-CD20 antibodies (e.g., rituximab (RITUXAN^{®})), anti-HER2/neu antibodies (e.g., trastuzumab (HERCEPTIN^{®})), alemtuxumab (CAMPATH^{®}), anti-EGFR antibodies (e.g., cetuximab, panitumumab), anti-VEGF antibodies (e.g., bevacizumab (AVASTIN^{®})); antibody conjugates, such as antibody drug conjugates (ADCs), Immune-stimulating Antibody Conjugates (ISACs), and antibody-oligonucleotide conjugates (AOCs). In some embodiments, the antibody conjugates are ADC class drugs. Non-limiting examples of ADCs include: Her2-ADC, Trop2-ADC, CD22-ADC, BCMA-ADC, CD19-ADC, Nectin-4-ADC.

In some embodiments, the second therapeutic agent is a chemotherapeutic agent. Non-limiting examples of chemotherapeutic agents include: alkylating agents such as thiotepa and CYTOXAN^{®} cyclosphosphamide; temozolomide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CB 1-TM1); eleutherobin; pancratistatin; a sarcodictyin; spongistatin; nitrogen mustards such as chlorambucil, chlomaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e.g., calicheamicin, especially calicheamicin gammall and calicheamicin omegal 1 (see, e.g., Agnew, Chem. Intl. Ed. Engl., 33: 183-186 ( 1994)); dynemicin, including dynemicin A; bisphosphonates, such as clodronate; an esperamicin; as well as neocarzinostatin chromophore and related chromoprotein enediyne antiobiotic chromophores), aclacinomysins, actinomycin, authramycin, azaserine, bleomycins, cactinomycin, carabicin, caminomycin, carzinophilin, chromomycinis, dactinomycin, daunorubicin, detorubicin, 6-diazo-5- oxo-L-norleucine, ADRIAMYCIN^{®} doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elformithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine; PSK^{®} polysaccharide complex (JHS Natural Products, Eugene, Oreg.); razoxane; rhizoxin; sizofuran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., TAXOL^{®} paclitaxel (Bristol-Myers Squibb Oncology, Princeton, N.J.), ABRAXANE^{®} Cremophor-free, albumin-engineered nanoparticle formulation of paclitaxel (American Pharmaceutical Partners, Schaumberg, 111.), and TAXOTERE^{®} doxetaxel (Rhone- Poulenc Rorer, Antony, France); chloranbucil; GEMZAR^{®} gemcitabine; 6-thioguanine; mercaptopurine; methotrexate; platinum analogs such as cisplatin, oxaliplatin and carboplatin, vinblastine; platinum; etoposide (VP-16); ifosfamide; mitoxantrone; vincristine; NAVELBINE, vinorelbine; novantrone; teniposide; edatrexate; daunomycin; aminopterin; xeloda; ibandronate; irinotecan (Camptosar, CPT-11) (including the treatment regimen of irinotecan with 5-FU and leucovorin); topoisomerase inhibitor RFS 2000; difluoromethylomithine (DMFO); retinoids such as retinoic acid; capecitabine; combretastatin; leucovorin (LV); oxaliplatin, including the oxaliplatin treatment regimen (FOLFOX); lapatinib (TYKERB); inhibitors of PKC-alpha, Raf, H-Ras, EGFR (e.g., erlotinib (TARCEVA^{®})) and VEGF-A that reduce cell proliferation and pharmaceutically acceptable salts, acids or derivatives of any of the above.

The subjects of the present invention include birds, reptiles, mammals, etc. Preferably, the mammals include rodents and primates, and preferably, the primates include humans.

The scope of the diseases involved in the present invention includes but is not limited to tumors. Preferably, the tumors include: leukemia, lymphoma, myeloma, brain tumor, head and neck squamous cell cancer, non-small cell lung cancer, nasopharyngeal cancer, esophageal cancer, gastric cancer, pancreatic cancer, gallbladder cancer, liver cancer, colorectal cancer, breast cancer, ovarian cancer, cervical cancer, endometrial cancer, uterine sarcoma, prostate cancer, bladder cancer, renal cell cancer, melanoma, small cell lung cancer and bone cancer.

A pharmaceutically acceptable carrier refers to a pharmaceutical carrier commonly used in the pharmaceutical art, such as diluents, excipients and water, etc.; fillers such as starch, sucrose, lactose, microcrystalline cellulose, etc.; binders such as cellulose derivatives, alginates, gelatin and polyvinylpyrrolidone; wetting agents such as glycerin; disintegrating agents such as sodium carboxymethyl starch, hydroxypropyl cellulose, croscarmellose, agar, calcium carbonate and sodium hydrogencarbonate; absorption enhancers such as quaternary ammonium compounds; surfactants such as cetanol, sodium lauryl sulfate; adsorption carriers such as kaolinite and bentonite; lubricants such as talc, calcium and magnesium stearate, micronized silica gel and polyethylene glycol, etc.. In addition, other adjuvants such as flavoring agents and sweetening agents may also be added into the composition.

The present invention will be further illustrated with reference to the following non-limiting examples. Those skilled in the art would know that many modifications may be made to the present invention without departing from the spirit of the present invention, and such modifications also fall within the scope of the present invention.

The following experimental methods are all conventional methods unless otherwise specified, and all the experimental materials used are easily available from commercial companies unless otherwise specified. The various antibodies used in the following examples of the present invention are all standard antibodies commercially available.

### Examples

### Example 1. Construction of the vector for anti-PD-L1/anti-4-1BB heterodimeric antibody molecule

The XOGC expression vectors containing the heavy chain or light chain of the anti-human PD-L1 antibody were constructed, respectively. The nucleotide sequence of the light chain variable region is set forth in SEQ ID NO. 1, and the amino acid sequence thereof is set forth in SEQ ID NO: 2; the nucleotide sequence of the light chain constant region is set forth in SEQ ID NO. 3, and the amino acid sequence thereof is set forth in SEQ. ID NO: 4; the nucleotide sequence of the heavy chain variable region is set forth in SEQ ID NO. 5, and the amino acid sequence thereof is set forth in SEQ ID NO: 6; the nucleotide sequence of the heavy chain constant region is set forth in SEQ ID NO. 7, and the amino acid sequence thereof is set forth in SEQ ID NO:8. The light chain variable region and the light chain constant region, as well as the heavy chain variable region and the heavy chain constant region, were amplified individually by PCR. All the PCR reactions in the present application used the Phusion ultra-fidelity DNA polymerase (F-530L) from NEB. PCR primers were conventionally designed according to the principle of base complementation and the needs for restriction sites. Each reaction system consists of: H₂O 8.9µl, 5×Phusion ultra-fidelity DNA polymerase buffer 4µl, 1mM dNTPs 4µl, forward primer 1µl, reverse primer 1µl, Phusion ultra-fidelity DNA polymerase 0.1µl, the template 1µl. The PCR products of the variable and constant regions were electrophoresed on 1.5% agarose gel, and corresponding fragments were recovered using a DNA recovery kit (Promega, A9282, the same hereinafter). Then, another round of PCR reaction with the recovered variable region fragments and constant region fragments as template was performed using the forward primer of the variable region and the reverse primer of the constant region, to obtain full-length fragments of the light chain or the heavy chain. The XOGC vectors and the full-length fragments were digested with EcoRI (Thermo, catalog number FD0275) and HindIII (Thermo, catalog number FD0505), and the digestion reaction system was: 10× buffer 6 µl, 2 µl EcoRI and 2 µl HindIII, the full-length fragments obtained from gel recovery 40µl, H₂O 10µl, and the digestion system reaction was conducted at 37°C for 1 hour. The digested products were ligated using T4DNA ligase (Thermo, catalog number EL0011) (the same hereinafter), and the reaction system was: 10×ligase buffer 1µl, the ligase 0.5µl, the full-length fragments obtained from gel recovery 7.5µl, the XOGC vector obtained from gel recovery 1µl, and the ligation reaction was conducted at 22°C for 30min. The ligation products were transformed into *E. coli* competent cell DH5α (Tiangen, CB104, the same hereinafter). The XOGC expression vectors for the anti-human PD-L1 antibody heavy chain and light chain were obtained and used to express the anti-human PD-L1 antibody heavy chain and light chain in eukaryotic cells, respectively.

The XOGC expression vectors containing the sequences of the heavy chain and light chain variable regions of the anti-human 4-1BB antibody were also constructed in the present invention respectively. The nucleotide sequence of the light chain variable region is set forth in SEQ ID NO. 9, and the amino acid sequence thereof is set forth in SEQ ID NO: 10; the nucleotide sequence of the light chain constant region is set forth in SEQ ID NO. 3, and the amino acid sequence thereof is set forth in SEQ. ID NO: 4; the nucleotide sequence of the heavy chain variable region is set forth in SEQ ID NO. 11, and the amino acid sequence thereof is set forth in SEQ ID NO: 12; the nucleotide sequence of the heavy chain constant region is set forth in SEQ ID NO. 13, and the amino acid sequence thereof is set forth in SEQ ID NO: 14. The XOGC expression vectors for the anti-human 4-1BB antibody heavy chain and light chain were obtained and used to express the anti-human 4-1BB antibody heavy chain and light chain in eukaryotic cells, respectively.

### Example 2. Expression of the anti-PD-L1/anti-4-1BB heterodimeric antibody molecule

The expression vectors containing the heavy or light chains of the anti-human PD-L1 antibody were co-transfected into ExpiCHO cells (ExpiCHO^{™} Cells, catalog number A29127, invitrogen). In addition, the expression vectors containing the heavy or light chains of the anti-human 4-1BB antibody were also co-transfected into ExpiCHO cells. The cells were seeded at a seeding density of 3.5×10⁶ cells/mL the day before transfection. On the day of transfection, the cells were diluted with fresh ExpiCHO expression medium (ExpiCHO^{™} Expression Medium, catalog number A29100-01, invitrogen) at a dilution density of 6×10⁶ cells/mL. The plasmids were taken out according to the transfection volume to achieve a final concentration of 0.5 µg/ml. The plasmids were diluted to 4% of the transfection volume using OptiPRO^{™} SFM medium (OptiPRO^{™} SFM, catalog number 12309-019, invitrogen), and mixed homogeneously by inversion. The ExpiFectamine^{™} transfection reagent (ExpiFectamine^{™} CHO Transfection Kit, catalog number A29129, invitrogen) in an amount of 6.4 times of the plasmid amount was taken out and was diluted to 4% of the transfection volume using OptiPRO^{™} SFM medium, and mixed homogeneously by inversion. The diluted transfection reagent was added to the diluted plasmids, mixed gently, leaved at room temperature for 1 to 5 min, and slowly added dropwise into the cells. The cells were then placed into a cell incubator (8% CO₂ concentration), and incubated at 37°C for 20 hours on a shaker at 125rpm. The ExpiCHO^{™} Enhancer (ExpiFectamine^{™} CHO Transfection Kit, catalog number A29129, invitrogen) in an amount of 0.006 times of the transfection volume and the ExpiCHO^{™} Feed (ExpiCHO^{™} Feed, catalog number A29101-02, invitrogen) in an amount of 0.24 times of the transfection volume were slowly added into the cells. The cells were placed in a shaker at 125 rpm and incubated at 32°C. The supernatant of the cell culture was collected by centrifugation 10 days after transfection.

The expression levels were determined by a Protein A assay. A filtration step with 0.22µm filter membrane was performed to remove the precipitates before applying the chromatography column purification. This step was performed at 4°C.

### Example 3. Purification of the expression products of the anti-PD-L1/anti-4-1BB heterodimeric antibody molecule

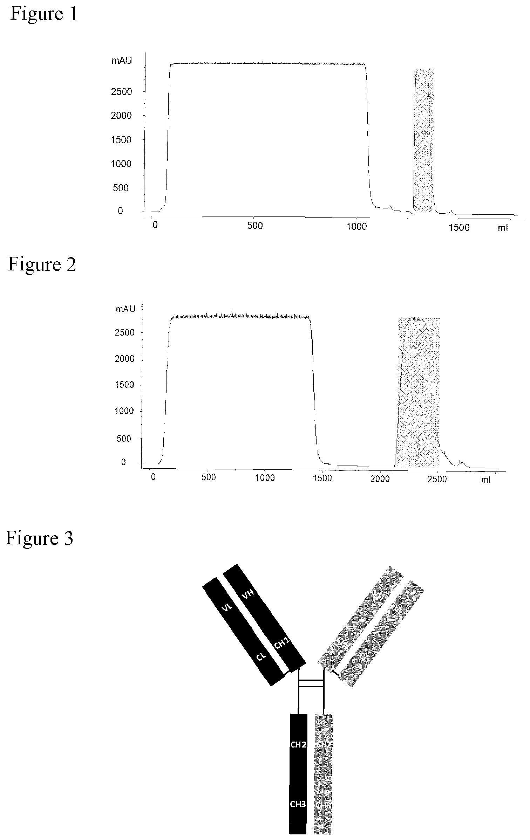

The purification was performed at room temperature using AKTA explorer 100 protein purification system (GE Healthcare) and affinity chromatography column MabSelect SuRe (GE Healthcare). The chromatography column was first equilibrated with mobile phase A (20 mM sodium phosphate buffer, pH 7.4), the cell supernatant as treated above was loaded once the baseline becomes stable, and then equilibrated with mobile phase A. The samples were the anti-PD-L1 expression products and the anti-4-1BB expression products, respectively. Thereafter, the column was firstly washed with 2-3 column volumes of mobile phase B (mobile phase A containing 1 M sodium chloride), and then washed with 2-3 column volumes of mobile phase A, and then eluted with 5 column volumes of mobile phase C (100 mM glycine, PH 3.5), and the elution peak collected was the target protein peak. The chromatogram of the elution peak of the anti-PD-L1 expression products is shown in Figure 1, and the elution peak of the anti-4-1BB expression products is shown in Figure 2. The elution peak marked (the gray area in the figure) was collected, adjusted to pH 7.2 by dropwise addition of 1 M Tris solution, adjusted to 0.15 M final concentration by adding NaCl, and sterile filtered and stored at 2~8°C.

### Example 4. Preparation and purification of the anti-PD-L1/anti-4-1BB heterodimeric antibody molecule

The structure of the anti-PD-L1/anti-4-1BB heterodimeric antibody molecule is shown in Figure 3.

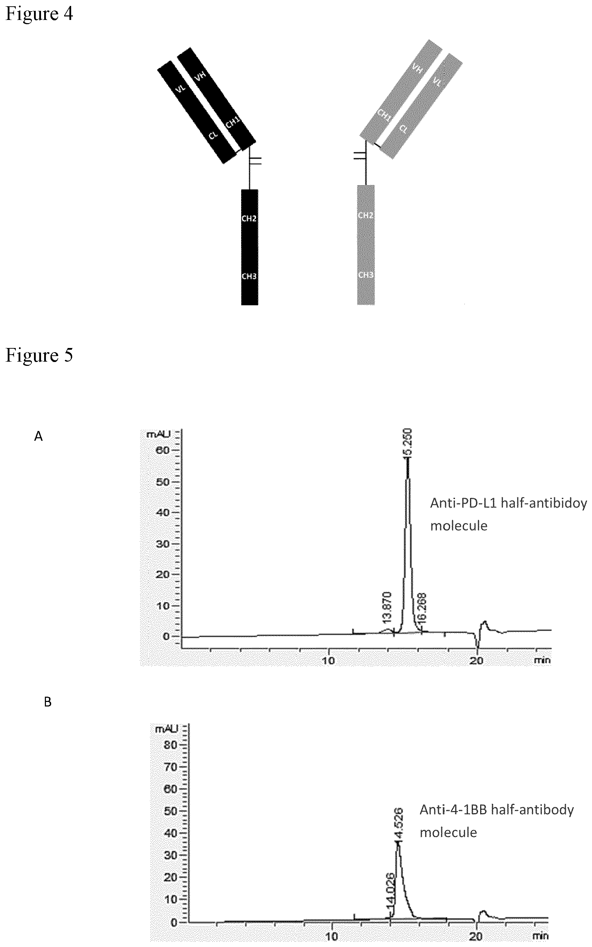

The products purified by the MabSelect SuRe (GE Healthcare) method described above were subjected to *in vitro* reconstitution to obtain heterodimers. First, the collected protein solutions after the purification described above were subjected to a cysteine reduction process to open up the disulfide bonds, and the disulfide bonds in the hinge region of the antibody homodimeric molecules contained in the anti-PD-L1 and anti-4-1BB products were also opened, to form half-antibody molecules containing one heavy chain and one light chain, of which the structure is shown in Figure 4. The reduced sample was analyzed by SEC-HPLC (shodex, protein kw-803) with mobile phase buffer containing 1mM DTT reducing agent. The results are shown in Figure 5A and 5B, respectively. The ratios of the anti-PD-L1 and the anti-4-1BB homodimeric molecules are both less than 10%, and the ratio of the half-antibody molecules is greater than 90%.

Next, the reduced anti-PD-L1 and anti-4-1BB half-antibody molecules were mixed in an equimolar ratio, and the reconstitution reaction was carried out at room temperature for 0.5 hours. During the reconstitution process, the anti-PD-L1 and the anti-4-1BB half-antibody molecules formed heterodimeric bispecific antibody containing both the anti-PD-L1 and the anti-4-1BB half-antibody molecules through non-covalent force between CH2 and CH3 domains. The protein solution was then subjected to ultrafiltration concentration (nominal molecular weight cut-off of 10KDa), and the solution was replaced with 20mM phosphate buffer, 0.15M NaCl, 0.1 mM cystine, and then incubated at room temperature overnight for oxidation reaction, to re-form the disulfide bonds in the heterodimeric bispecific antibody.

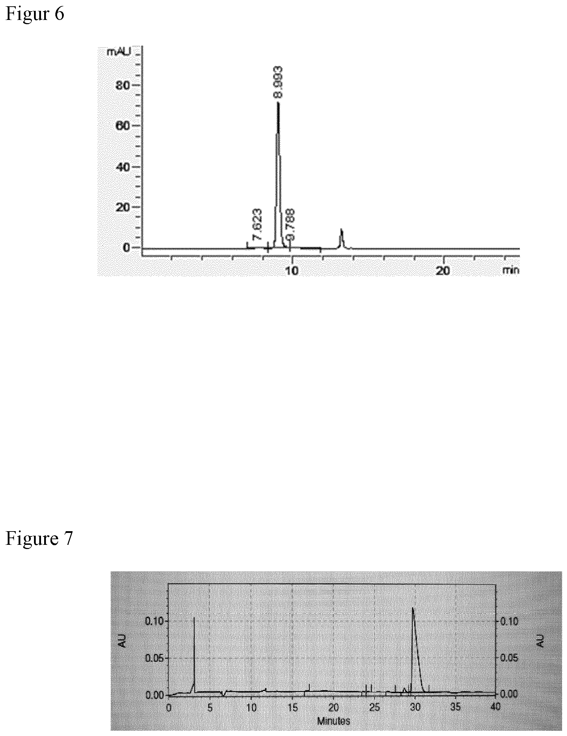

The heterodimeric antibody molecules obtained above by reduction and oxidation of the anti-PD-L1 and anti-4-1BB expression products were subjected to ultrafiltration concentration (nominal molecular weight cut-off of 10KDa), and the solution was replaced with 20 mM citrate buffer, pH 6.0. The purification was performed at room temperature using AKTA explorer 100 protein purification system (GE Healthcare) and ion chromatography column Poros XS (ThermoFisher). First, the chromatography column was equilibrated with mobile phase A (20 mM citric acid, pH 6.0). The protein solution as treated above was loaded once the baseline becomes stable, and then equilibrated with mobile phase A. Next, the column was washed with 15 column volumes of a gradient from A (20 mM Citric acid, pH 6.0) to B (20 mM citric acid, 200 mM arginine, pH 6.0) (0% B-100% B, 80 min). The main elution peak was collected, and the collected protein solution was subjected to ultrafiltration concentration (nominal molecular weight cut-off of 10KDa), and then the solution was replaced with 20 mM citric acid, 140 mM arginine, pH 6.0; and sterilized through filtration, and then stored at 4°C after adding 0.02% Tween80. The purity of the purified anti-PD-L1/anti-4-1BB heterodimeric antibody molecules BH3120h analyzed by SEC-HPLC was 98.54%, as shown in Figure 6; CE analysis was also performed, and the purity is 96.44%, as shown in Figure 7.

### Example 5. Target binding activity of the anti-PD-L1/anti-4-1BB heterodimeric antibody

Enzyme-linked immunosorbent assay (ELISA) was used to determine the binding ability of the anti-PD-L1/anti-4-1BB heterodimeric antibody to a single antigen. The detailed process is as follows: a 96-well high-adsorption ELISA plate (Costar, catalog number 42592) was coated with 1 µg/mL recombinant human PD-L1 (Sino Biological Inc., catalog number 100 84-H08H) or human 4-1BB (Sino Biological Inc., catalog number 10041-H08H) at 100 µL per well using carbonate buffer solution, pH=9.6 at 4°C overnight. The plate was wash 5 times with PBST, and then blocked using PBST with 1% BSA 300 µL/well and incubated at 25°C for 1 hour, and then washed 5 times with PBST. The heterodimeric antibody samples serially diluted in PBST with 1% BSA and the control were added, 100 µL per well and incubated at 25°C for 1 hour. The plate was washed 5 times with PBST. The horseradish peroxidase-labeled anti-human IgG antibody (Chemicon, catalog number AP309P) diluted 1:10000 in PBST with 1% BSA was then added, 100 µL per well, and incubated at 25°C for 1 hour. The plate was washed 5 times with PBST. The colorimetric substrate TMB was added, 100µL/well, and developed at room temperature for 10 minutes. 1M H₂SO₄, was added, 100µL/well, to stop the development. The absorbance at 450nm was read on a microplate reader.

Meanwhile, GS-H2/4-1BB cells were used to determine the binding ability of the anti-PD-L1/anti-4-1BB heterodimeric antibody to 4-1BB antigen. The detailed process is as follows: the GS-H2/4-1BB cells (purchased from GenScript) were collected and washed once with cold DPBS (GIBCO, catalog number 14190-136) containing 2% FBS (Hyclone, catalog number SH30084.03). GS-H2/4-1BB cells were re-suspended in cold DPBS with 2% FBS at a density of 5×10⁶ cells/mL. 100µL of the cell suspension was added to each test tube, as well as 100µL of the serially diluted heterodimeric antibody sample and the control. The test tubes were incubated on ice for 30 minutes, washed twice with DPBS containing 2% FBS, and re-suspended in 200 µL cold DPBS of 488A-Fab-anti-human IgG (final concentration 5 µg/mL) containing 2% FBS. The tubes were then incubated on ice for 30 minutes keeping from light, washed twice with DPBS containing 2% FBS, and re-suspended again in 500 µL cold DPBS. The cell suspension was detected and analyzed on a flow cytometer (BD, FACS Calibur), and the fluorescence intensity of the cells was read.

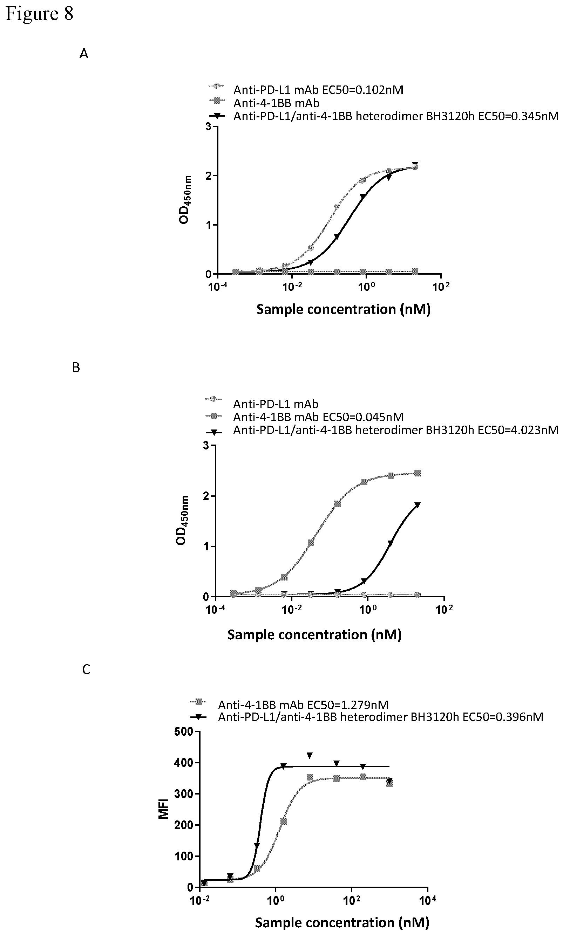

As shown by the results of Figure 8A, the anti-PD-L1/anti-4-1BB heterodimeric antibody BH3120h has a high affinity with PD-L1, which is slightly lower than the antigen binding activity of the PD-L1 bivalent monoclonal antibody. As shown in Figure 8B, the affinity of the anti-PD-L1/anti-4-1BB heterodimeric antibody BH3120h with 4-1BB is relatively low, which is lower than the antigen binding activity of the 4-1BB bivalent monoclonal antibody. As shown in Figure 8C, the affinity of the anti-PD-L1/anti-4-1BB heterodimeric antibody BH3120h with 4-1BB is relatively high, which is higher than the antigen-binding activity of the anti-4-1BB bivalent monoclonal antibody.

### Example 6. Ligand-receptor binding blocking activity of the anti-PD-L1/anti-4-1BB heterodimeric antibody

The detection process of the blocking activity for PD-L1 binding to PD-1 and CD80 is as follows: a 96-well high-binding ELISA plate was coated with 1 µg/mL human PD-L1-Fc (ACROBiosystems, catalog number PD1-H5258), 100 µL per well, using carbonate buffer solution, pH=9.6 at 4°C overnight, and washed 5 times with PBST. The plate was then blocked with PBST containing 1% BSA, 300 µL/well, incubated at 25°C for 1 hour, and washed 5 times with PBST. The heterodimeric antibody samples serially diluted in PBST with 1% BSA and the control were added, 50µL per well, as well as 50µL of the biotin-labeled PD-1-Fc (Beijing Hanmi Pharmaceutical Co., LTD.) at a concentration of 40nM (final concentration 20nM) or 50µL of the biotin-labeled CD80-Fc (ACROBiosystems, catalog number B71-H82F2) at a concentration of 100nM (final concentration 50nM), and the plate was incubated at 25°C for 90 minutes and washed 5 times with PBST. Streptavidin-HRP (BD Pharmingen, catalog number 554066) diluted 1:1000 in PBST with 1% BSA was then added, 100µL per well, and the plate was incubated at 25°C for 1 hour and washed 5 times with PBST. The colorimetric substrate TMB was added, 100µL/well, and developed at room temperature for 10 minutes. 1M H₂SO₄ was then added, 100µL/well, to stop the development. The absorbance at 450nm was read on a microplate reader.

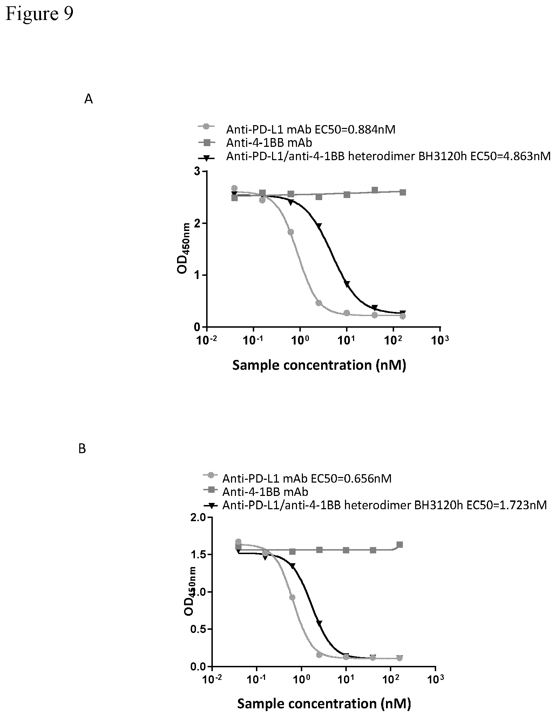

As shown by the result of Figure 9A, the anti-PD-L1/anti-4-1BB heterodimeric antibody BH3120h is able to block the binding of PD-L1 to PD-1, with the activity slightly weaker than that of the anti-PD-L1 bivalent monoclonal antibody. As shown in Figure 9B, the anti-PD-L1/anti4-1BB heterodimeric antibody BH3120h is able to block the binding of PD-L1 to CD80.

### Example 7. T cell modulation activity of the anti-PD-L1/anti-4-1BB heterodimeric antibody

A mixed lymphocyte reaction (MLR) was used to determine the modulation activity of the anti-PD-L1/anti-4-1BB heterodimeric antibody on T cell immune response.

Obtaining of the human dendritic cells (DCs): the human monocytes were isolated according to the instructions of the Monocyte Cell Isolation Kit (Miltenyi Biotech, catalog number 130091153). Briefly, PBMCs (Allcells, catalog number PB0005-C) were washed once with DPBS (GIBCO, catalog number 14190-136) and then re-suspended at 10⁷ cells per 40µL separation buffer (PBS with 2mM EDTA, 0.5% BSA, pH=7.2) (the following amounts used are all for 10⁷ cells), and 10µL FcR blocking reagent and 10µL Biotin Antibody Cocktail were added and incubated at 4°C for 5 minutes. 30µL separation buffer and 20µL Anti-Biotin MicroBeads were then added and incubated at 4°C for 10 minutes. The human monocytes were obtained after passing the MACS separation column. The human monocytes were re-suspended in serum-free RPMI 1640 medium (GIBCO, catalog number 22400-089) at a cell density of 5×10⁶/mL and seeded in a cell culture flask and cultured in a complete medium (RPMI 1640 with 10% FBS) with the addition of 200ng/ml GM-CSF (Sino Biological Inc., catalog number 10015-HNAH) and 100ng/ml IL-4 (Sino Biological Inc., catalog number 11846-HNAE). The cells were incubated for 3 days, and the medium was replaced, and incubated for another 3 days. Then the medium was replaced with a complete medium (RPMI 1640 with 10% FBS) containing 200ng/ml GM-CSF, 100ng/ml IL-4 and 20ng/ml T`NF-α (Sino Biological Inc., catalog number 10602-HNAE), and incubated for one day to obtain the DCs.

Obtaining of the human T cells: the human PBMC cells were recovered and collected to ensure that these PBMCs and the PBMCs for inducing the DC cells are from different individuals. The human T cells were isolated according to the instructions of the Pan T cell isolation kit (Miltenyi Biotech, catalog number 130096535). Briefly, PBMCs were washed once with DPBS, and then re-suspended at 10⁷ cells per 40µL separation buffer (PBS with 2mM EDTA, 0.5% BSA, pH=7.2) (the following amounts used are all for 10⁷ cells). 10µL Pan T cell Biotin Antibody Cocktail was added and incubated at 4°C for 5 minutes. 30µL separation buffer and 20µL Pan T cell MicroBead Cocktail were then added and incubated at 4°C for 10 minutes. The T cells were obtained after passing the MACS separation column.

The collected human DCs and human T cells were re-suspended in a complete medium (RPMI 1640 with 10% FBS) and seeded in a 96-well plate at 1×10⁴ cells/well and at 1×10⁵ cells/well, respectively, mixed and incubated. The heterodimeric antibody samples serially diluted with a complete medium and the control were added. The culture plate was placed in a CO₂ incubator at 37°C and incubated for 5 days. After the incubation, the supernatants were removed from the wells and the cytokine contents were detected using the IL-2 detection kit (RayBiotech, catalog number ELH-IL2) according to the instructions thereof. Briefly, 100µL of the standard and diluted samples were added into the sample detection plate and incubated at 25°C for 2.5 hours. The plate was washed 5 times with PBST. 100µL of biotin-labeled detection antibody was then added and incubated at 25°C for 1 hour. The plate was washed 5 times with PBST. Horseradish peroxidase-labeled streptavidin was then added, 100µL/well, and incubated at 25°C for 45 minutes. The plate was washed 5 times with PBST. The colorimetric substrate TMB was added, 100µL/well, and developed at room temperature for 10 minutes. 1M H₂SO₄ was then added ,100pL/well, to stop the development. The absorbance at 450nm was read on a microplate reader.

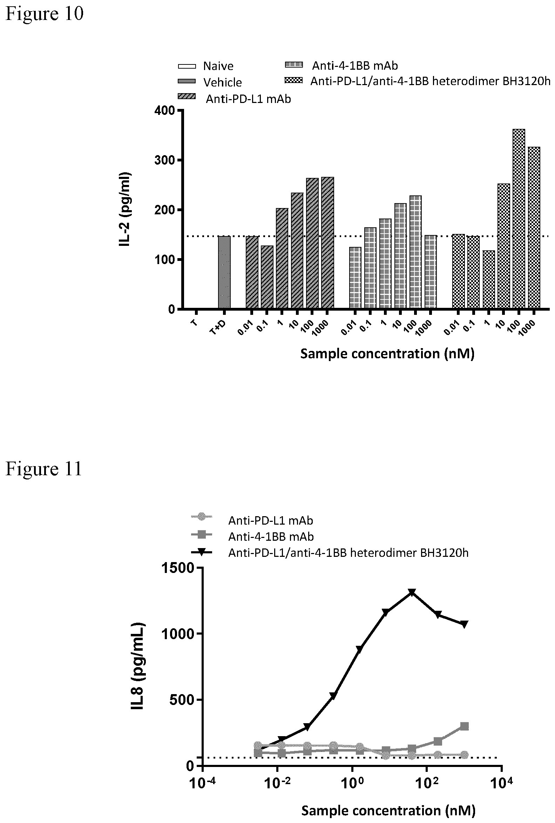

As shown by the results of Figure 10, the human T cells can secrete IL-2under the stimulation of allogeneic DCs. The additions of the anti-PD-L1 and anti-4-1BB bivalent antibodies can enhance the T cell activation and promote the secretion of cytokines. The anti-PD-L1/anti-4-1BB heterodimeric antibody BH3120h also showed very strong T cell modulation activity and significantly promoted the secretion of the cytokine IL-2.

### Example 8. The 4-1BB agonistic activity mediated by the anti-PD-L1/anti-4-1BB heterodimeric antibody

The GS-H2/4-1BB cells (purchased from GenScript) were collected and washed once with MEM+Glu medium (GIBCO, catalog number 41090101) with 2% FBS (Hyclone, catalog number SH30084.03). The GS-H2/4-1BB cells were re-suspended in the MEM+Glu medium with 2% FBS at a density of 1×10⁵ cells/mL. The cell suspension was added into a 96-well flat-bottom cell culture plate (Costar, catalog number 3599), 100 µL per well, and then placed in a CO₂ incubator at 37°C for 2 hours to allow the cells to adhere to the culture plate. The heterodimeric antibody samples and the control were diluted in the MEM+Glu medium with 2% FBS, from which 50 µL was taken out and added to the cell culture plate with GS-H2/4-1BB cells seeded. Meanwhile, DLD-1/PD-L1 cells (DLD-1 was purchased from Chinese Academy of Sciences) were collected and used to prepare the cell suspension at 1×10⁵ cells/mL using the MEM+Glu medium with 2% FBS, from which 50µL was then taken out and added to the cell culture plate with the heterodimeric antibody samples and the control added and with the GS-H2/4-1BB cells seeded. The cell culture plate was placed in a CO₂ incubator at 37°C for 24 hours. The cell culture supernatants thereof were taken out, and the cytokine content thereof was detected using the IL-8 detection kit (Dakewe Biotech Co., Ltd., catalog number 1110802) according to the instructions thereof. Briefly, 100µL of the standard and diluted samples were added into the sample detection plate, as well as 50µL of biotin-labeled detection antibody, incubated at 25°C for 1 hour and washed 5 times with PBST. Horseradish Peroxidase-labeled streptavidin was then added, 100µL/well, and the plate was incubated for 1 hour at 25°C and washed 5 times with PBST. The colorimetric substrate TMB was then added, 100µL/well, and the plate was developed at room temperature for 10 minutes. 1M H₂SO₄ was added, 100µL/well, to stop the development. The absorbance at 450nm was read on a microplate reader.

As shown by the results of Figure 11, the addition of the anti-4-1BB bivalent monoclonal antibody can mediate the activation of GS-H2/4-1BB cells to a certain extent and a small amount of IL-8 secretion. The addition of anti-PD-L1 bivalent antibody cannot activate the GS-H2/4-1BB cells. While the anti-PD-L1/anti-4-1BB heterodimeric antibody BH3120h can crosslink PD-L1 on the DLD-1/PD-L1 cells with 4-1BB on the GS-H2/4-1BB cells, resulting in the aggregation of 4-1BB and the strong activation of the GS-H2/4-1BB cells, as well as the production of a large amount of IL-8.

### Example 9. Efficacy study of the anti-PD-L1/anti-4-1BB heterodimeric antibody against MC38 tumor in gene knock-in mice

The 6-8-week-old female hPD-L1/h4-1BB dual humanized mice (in which the mouse-derived PD-L1 and 4-1BB were knocked out, and the human-derived PD-L1 and 4-1BB were overexpressed, Biocytogen, Jiangsu) were selected as the experimental material. One week after the mice have adapted to the environment, each mouse was subcutaneously inoculated with 5×10⁵ MC38/hPD-L1 mouse colon tumor cells (MC38 was purchased from Shunran Biology, Shanghai) on the right back. Once the tumors grow to a volume of about 100 mm³, the mice were divided into different groups according to the tumor volumes, 6 tumor-bearing mice per group. The vehicle (DPBS, GIBCO, catalog number 14190-136), the anti-PD-L1 monoclonal antibody 35nmol/kg, the anti-4-1BB monoclonal antibody 35nmol/kg and the anti-PD-L1/anti4-1BB heterodimeric antibody 70nmol /kg (allowing for the fact that the monoclonal antibody is a bivalent antibody, and the anti-PD-L1 and anti-4-1BB activities of the bispecific antibody are both monovalent) were administered respectively, 3 times a week for 2 consecutive weeks, via an intraperitoneal injection. The tumor volumes were measured twice a week beginning at the day of administration, and the long diameter a and the short diameter b were measured and the tumor volume calculation formula is: tumor volume (mm3) = (a × b²)/2. The tumor volume measurement lasted for 4 weeks, i.e., the observation was continued for another 2 weeks after the medication has been stopped.

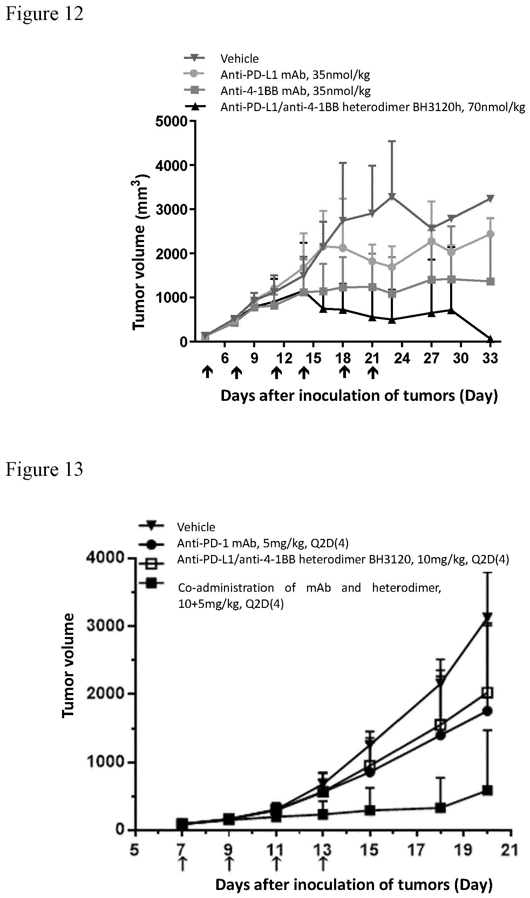

As shown by the results of Figure 12, the anti-PD-L1/anti-4-1BB heterodimeric antibody BH3120h has a stronger anti-tumor efficacy than the anti-PD-L1 bivalent antibody and the anti-4-1BB bivalent antibody in the homologous tumor model.

### Example 10. Efficacy study of co-administration of the anti-PD-L1/anti-4-1BB heterodimeric antibody and the anti-PD-1 antibody against CT-26 tumor in knock-in mice

The 6-8-week-old female hPD-1/hPD-L1/hCD137 triple knock-in mice (Gempharmatech Co., Ltd, Jiangsu) were selected as the experimental material. One week after the mice have adapted to the environment, each mouse was subcutaneously inoculated with 5×10⁵ CT-26/hPD-L1 mouse colon tumor cells (Gempharmatech Co., Ltd, Jiangsu) on the right back. Once the tumors grow to a volume of about 100 mm³, the mice were divided into different groups according to the tumor volumes randomly. The vehicle (DPBS), the anti-PD-L1/anti4-1BB heterodimeric antibody 10mg/kg, the anti-PD-1 monoclonal antibody (BH2917b) 5mg/kg and the co-administration group (10 +5mg/kg) were administered respectively, once every two days for 4 times, via an intraperitoneal injection. The tumor volumes were measured three times a week beginning at the day of administration, and the long diameter and the short diameter were measured and the tumor volume calculation formula is: tumor volume = [(short diameter² × long diameter)/2]. The inhibition rate of the tumor growth was calculated as follows: inhibition rate of tumor growth = [1-RTV (experimental group)/RTV (control group)] × 100%. RTV: relative tumor volume, RTV = VₜV₀, Vₜ: tumor volume at time t, Va: tumor volume at the beginning. As shown by the results of Figure 13, the co-administration of the anti-PD-L1/anti-4-1BB heterodimeric antibody BH3120 and the anti-PD-1 antibody shows a stronger, synergistic anti-tumor efficacy in the homologous tumor model. 20 days after inoculating CT-26-hPD-L1 cells, both the tumor volumes for BH2917b (5mg/kg) and BH3120 (10mg/kg) decreased as compared to the control group, with 44.1% and 37.3% inhibition rates of tumor growth respectively. BH3120 + BH2917b (10 + 5mg/kg) can significantly inhibit the tumor proliferation, with a 79.6% inhibition rate of tumor growth. Analyzing with a t test and compared to BH3120 group (10mg/kg), the tumor volume for the co-administration group decreased apparently and the difference is statistically significant (p <0.05); compared to BH2917b group (5mg/kg), the tumor volume for the co-administration group decreased but the difference is not statistically significant (p >0.05).

### Example 11. Efficacy study of co-administration of the anti-PD-L1/anti-4-1BB heterodimeric antibody and the anti-PD-1 antibody against MC38 tumor in knock-in mice

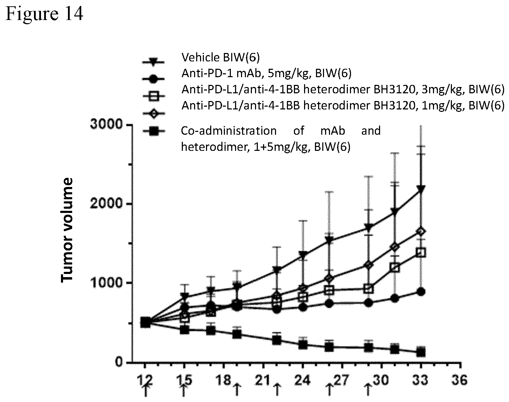

The 6-8-week-old female hPD-1/hCD137 double knock-in mice (Gempharmatech Co., Ltd and Biocytogen, Jiangsu) were selected as the experimental material. One week after the mice have adapted to the environment, each mouse was subcutaneously inoculated with 1×10⁶ MC38/hPD-L1 mouse colon tumor cells (Gempharmatech Co., Ltd, Jiangsu) on the right back. Once the tumors grow to a volume of about 100 mm³, the mice were divided into different groups according to the tumor volumes randomly. The vehicle (DPBS), the anti-PD-L1/anti4-1BB heterodimeric antibody 1mg/kg and 3mg/kg, the anti-PD-1 monoclonal antibody (BH2917b) 5mg/kg and the co-administration group (1 +5mg/kg) were administered respectively, 2 times a week for 6 times, via an intraperitoneal injection. The tumor volumes were measured three times a week beginning at the day of administration, and the long diameter and the short diameter were measured and the tumor volume calculation formula is: tumor volume = [(short diameter² × long diameter)/2]. The inhibition rate of the tumor growth was calculated as follows: inhibition rate of tumor growth = [1-RTV (experimental group)/RTV (control group)] × 100%. RTV: relative tumor volume, RTV = Vₜ/V₀, Vₜ: tumor volume at time t, Va: tumor volume at the beginning. As shown by the results of Figure 14, the co-administration of the anti-PD-L1/anti-4-1BB heterodimeric antibody BH3120 and the anti-PD-1 antibody shows a stronger, synergistic anti-tumor efficacy in the homologous tumor model. 33 days after inoculating hPD-L1/MC38 cells, the groups of administration of a single antibody showed a certain inhibition effect on the tumor with a dose-dependent mode, with inhibition rates of tumor growth as follows: BH2917b (5 mg/kg) 76.58 %, BH3120 (3 mg/kg) 47.07 %, BH3120 (1 mg/kg) 30.95 %, respectively. Co-administration of BH3120 + BH2917b (1+5 mg/kg) can inhibit the tumor proliferation significantly, with a 122.40% TGI_{TV}%. Analyzing with a t test and compared to BH3120 group (1mg/kg), the difference is statistically significant (p <0.05); compared to BH2917b group (5mg/kg), the difference is statistically significant (p <0.01).

## Claims

1. A bispecific antibody comprising a first antigen-binding functional region that specifically binds to PD-L1 and a second antigen-binding functional region that specifically binds to 4-1BB, wherein
the first antigen-binding functional region that specifically binds PD-L1 comprises:
(A) a heavy chain variable region, comprising
(a) HCDR1, which comprises the amino acid sequence set forth in SEQ ID NO: 15,
(b) HCDR2, which comprises the amino acid sequence set forth in SEQ ID NO: 16, and
(c) HCDR3, which comprises the amino acid sequence set forth in SEQ ID NO: 17; and
(B) a light chain variable region, comprising
(a) LCDR1, which comprises the amino acid sequence set forth in SEQ ID NO: 18,
(b) LCDR2, which comprises the amino acid sequence set forth in SEQ ID NO: 19, and
(c) LCDR3, which comprises the amino acid sequence set forth in SEQ ID NO: 20.

2. The bispecific antibody of claim 1, wherein
the second antigen-binding functional region that specifically binds to 4-1BB comprises
(A) a heavy chain variable region, comprising
(a) HCDR1, which comprises the amino acid sequence set forth in SEQ ID NO: 21,
(b) HCDR2, which comprises the amino acid sequence set forth in SEQ ID NO: 22, and
(c) HCDR3, which comprises the amino acid sequence set forth in SEQ ID NO: 23; and
(B) a light chain variable region, comprising
(a) LCDR1, which comprises the amino acid sequence set forth in SEQ ID NO: 24,
(b) LCDR2, which comprises the amino acid sequence set forth in SEQ ID NO: 25, and
(c) LCDR3, which comprises the amino acid sequence set forth in SEQ ID NO: 26.

3. The bispecific antibody of claim 1 or claim 2, wherein
the first antigen-binding functional region that specifically binds to PD-L1 comprises
(A) a heavy chain variable region, comprising
(a) HCDR1, which comprises the amino acid sequence set forth in SEQ ID NO: 15,
(b) HCDR2, which comprises the amino acid sequence set forth in SEQ ID NO: 16, and
(c) HCDR3, which comprises the amino acid sequence set forth in SEQ ID NO: 17; and
(B) a light chain variable region, comprising
(a) LCDR1, which comprises the amino acid sequence set forth in SEQ ID NO: 18,
(b) LCDR2, which comprises the amino acid sequence set forth in SEQ ID NO: 19, and
(c) LCDR3, which comprises the amino acid sequence set forth in SEQ ID NO: 20; and
the second antigen-binding functional region that specifically binds to 4-1BB comprises
(A) a heavy chain variable region, comprising
(a) HCDR1, which comprises the amino acid sequence set forth in SEQ ID NO: 21,
(b) HCDR2, which comprises the amino acid sequence set forth in SEQ ID NO: 22, and
(c) HCDR3, which comprises the amino acid sequence set forth in SEQ ID NO: 23; and
(B) a light chain variable region, comprising
(a) LCDR1, which comprises the amino acid sequence set forth in SEQ ID NO: 24,
(b) LCDR2, which comprises the amino acid sequence set forth in SEQ ID NO: 25, and
(c) LCDR3, which comprises the amino acid sequence set forth in SEQ ID NO: 26.

4. The bispecific antibody of any one of claims 1-3, wherein the first antigen-binding functional region that specifically binds to PD-L1 comprises a heavy chain variable region comprising the amino acid sequence set forth in SEQ ID NO: 6, and a light chain variable region comprising the amino acid sequence set forth in SEQ ID NO: 2.

5. The bispecific antibody of any one of claims 1-4, wherein the second antigen-binding functional region that specifically binds to 4-1BB comprises a heavy chain variable region comprising the amino acid sequence set forth in SEQ ID NO: 12, and a light chain variable region comprising the amino acid sequence set forth in SEQ ID NO: 10.

6. The bispecific antibody of any one of claims 1-5,
wherein the first antigen-binding functional region that specifically binds to PD-L1 comprises a heavy chain variable region comprising the amino acid sequence set forth in SEQ ID NO: 6, and a light chain variable region comprising the amino acid sequence set forth in SEQ ID NO: 2; and
wherein the second antigen-binding functional region that specifically binds to 4-1BB comprises a heavy chain variable region comprising the amino acid sequence set forth in SEQ ID NO: 12, and a light chain variable region comprising the amino acid sequence set forth in SEQ ID NO: 10.

7. The bispecific antibody of any one of claims 1-6, wherein the first antigen-binding functional region and the second antigen-binding functional region are selected from the group consisting of Fab fragments, scFv fragments, and variable domain fragments Fv.

8. The bispecific antibody of any one of claims 1-7, wherein the first antigen-binding functional region and the second antigen-binding functional region are both Fab fragments.

9. The bispecific antibody of any one of claims 1-8, wherein the Fab fragment thereof comprises a different first heavy chain variable region and a different second heavy chain variable region, as well as a different first light chain variable region and a different second light chain variable region.

10. The bispecific antibody of any one of claims 1-9, wherein one of the first antigen-binding functional region and the second antigen-binding functional region is a Fab fragment, and the other is a scFv.

11. The bispecific antibody of claims 1-10, comprising a first Fc chain and a second Fc chain,
wherein the first Fc chain and the second Fc chain are both immunoglobulin G Fc fragments containing amino acid substitutions, and the first Fc chain and the second Fc chain together constitute a heterodimer that can bind to the Fc receptor;
wherein the first Fc chain and the second Fc chain are linked to the first antigen-binding functional region and the second antigen-binding functional region, respectively, via a covalent bond or a linker; and
wherein either the first Fc chain or the second Fc chain comprises T366L and D399R amino acid substitutions at positions 366 and 399, and the other comprises L351E, Y407L and K409V amino acid substitutions at positions 351, 407 and 409, wherein the amino acid positions are numbered according to the Kabat EU index numbering system.

12. The bispecific antibody of any one of claims 1-11, wherein the weight ratios of the homodimers formed by the first Fc chain and the first antigen-binding functional region covalently linked thereto and by the second Fc chain and the second antigen -binding functional region covalently linked thereto of said bispecific antibody are less than 50% of the total amount of all the polypeptide chains, in a reducing agent-containing solution in which no other polypeptides present except for said Fc chains and the antigen-binding functional regions.

13. The bispecific antibody of claims 1-12, comprising
a first heavy chain/first light chain pair that specifically binds to PD-L1, wherein the first heavy chain has a heavy chain variable region comprising the amino acid sequence set forth in SEQ ID NO: 6, and a heavy chain constant region comprising the amino acid sequence set forth in SEQ ID NO: 8; the first light chain has a light chain variable region comprising the amino acid sequence set forth in SEQ ID NO: 2, and a light chain constant region comprising the amino acid sequence set forth in SEQ ID NO: 4.

14. The bispecific antibody of claims 1-13, comprising
a second heavy chain/second light chain pair that specifically binds to 4-1BB, wherein the second heavy chain has a heavy chain variable region comprising the amino acid sequence set forth in SEQ ID NO: 12, and a heavy chain constant region comprising the amino acid sequence set forth in SEQ ID NO: 14; the second light chain has a light chain variable region comprising the amino acid sequence set forth in SEQ ID NO: 10, and a light chain constant region comprising the amino acid sequence set forth in SEQ ID NO: 4.

15. An isolated polynucleotide encoding the bispecific antibody of any one of claims 1-14.

16. A recombinant expression vector comprising the isolated polynucleotide of claim 15.

17. A host cell comprising the isolated polynucleotide of claim 15 or the recombinant expression vector of claim 16.

18. The host cell of claim 17, selected from the group consisting of human embryonic kidney cells HEK293, or HEK293T, HEK293E, HEK293F that are modified from HEK293 cells; hamster ovary cells CHO, or CHO-S, CHO-dhfr-, CHO/DG44, ExpiCHO that are modified from CHO cells; *Escherichia coli,* or *Escherichia coli* BL21, BL21(DE3), Rosetta, Origami that are modified from *E. coli*; Yeast, or *Pichia, Saccharomyces cerevisiae, Kluyveromyces lactis, Hansenula polymorpha* that are modified from Yeast; insect cells, or High5, SF9 cells that are modified from insect cells; plant cells; mammalian mammary gland cells; somatic cells.

19. A composition comprising the bispecific antibody of any one of claims 1-14 or the isolated polynucleotide of claim 15 or the recombinant expression vector of claim 16 or the host cell of claim 17 or 18, and a pharmaceutically acceptable carrier.

20. The composition of claim 19, further comprising a second therapeutic agent, preferably, the second therapeutic agent and the bispecific antibody, the isolated polynucleotide, the recombinant expression vector or the host cell are located in different parts of the composition, and preferably, the second therapeutic agent is selected from the group consisting of a second antibody, an immunotherapeutic agent, a targeted therapeutic agent or a chemotherapeutic agent, preferably, the second therapeutic agent is an anti-PD-1 antibody and/or a STING agonist.

21. A method of producing the bispecific antibody of any one of claims 1-14, including the steps of:
1) Expressing the isolated polynucleotide of claim 15 or the recombinant expression vector of claim 16 in host cells, respectively;
2) Reducing the proteins respectively expressed in the host cells; and
3) Mixing the reduced proteins, and then oxidizing the mixture.

22. The method of claim 21, wherein the reduction step includes 1) performing the reduction reaction in the presence of a reducing agent selected from the group consisting of 2-mercaptoethylamine, dithiothreitol, tris(2-carboxyethyl) phosphine or other chemical derivatives thereof; 2) Removing the reducing agent.

23. The method of claim 21 or 22, wherein the oxidation step is conducted in the air or in the presence of an oxidizing agent selected from the group consisting of L-dehydroascorbic acid or chemical derivatives thereof.

24. The method of any one of claims 21-23, further including a step of separation and purification.

25. Use of the bispecific antibody of any one of claims 1-14 and/or the isolated polynucleotide of claim 15 and/or the recombinant expression vector of claim 16 and/or the host cell of claim 17 or 18 and/or the composition of claim 19 or 20, in the manufacture of a medicament for preventing and/or treating diseases in a subject.

26. The bispecific antibody of any one of claims 1-14 and/or the isolated polynucleotide of claim 15 and/or the recombinant expression vector of claim 16 and/or the host cell of claim 17 or 18 and/or the composition of claim 19 or 20, for use as a medicament for preventing and/or treating diseases in a subject.

27. A method of preventing and/or treating diseases, including administering to a subject in need thereof the bispecific antibody in a heterodimeric form of any one of claims 1-14 and/or the isolated bispecific antibody of claim 15 and/or the recombinant expression vector of claim 16 and/or the host cell of claim 17 or 18 and/or the composition of claim 19 or 20.

28. Use of claim 25, the bispecific antibody in a heterodimeric form, the isolated polynucleotide, the recombinant expression vector, the host cell or the composition of claim 26, or the method of claim 27, wherein the subject is a mammal, preferably a human subject.

29. Use of claim 25, the bispecific antibody in a heterodimeric form, the isolated polynucleotide, the recombinant expression vector, the host cell or the composition of claim 26, or the method of claim 27, wherein the diseases are selected from the group consisting of the following tumors: leukemia, lymphoma, myeloma, brain tumor, head and neck squamous cell cancer, non-small cell lung cancer, nasopharyngeal cancer, esophageal cancer, gastric cancer, pancreatic cancer, gallbladder cancer, liver cancer, colorectal cancer, breast cancer, ovarian cancer, cervical cancer, endometrial cancer, uterine sarcoma, prostate cancer, bladder cancer, renal cell cancer, melanoma, small cell lung cancer and bone cancer.
